(19) Europäisches Patentamt
European Patent Office
Office européen des brevets

(11) **EP 3 746 043 B1**

(12) **EUROPEAN PATENT SPECIFICATION**

(45) Date of publication and mention
of the grant of the patent:
**23.10.2024 Bulletin 2024/43**

(21) Application number: **19743182.8**

(22) Date of filing: **28.01.2019**

(51) International Patent Classification (IPC):
*A61K 8/9789* (2017.01)    *A61K 8/60* (2006.01)
*A61K 8/99* (2017.01)    *A61K 8/06* (2006.01)
*A61Q 19/08* (2006.01)

(52) Cooperative Patent Classification (CPC):
**A61K 8/9789; A61K 8/60; A61K 8/99; A61Q 19/08;**
A61Q 17/04

(86) International application number:
**PCT/US2019/015377**

(87) International publication number:
**WO 2019/148080 (01.08.2019 Gazette 2019/31)**

(54) **TOPICAL COMPOSITIONS**

TOPISCHE ZUSAMMENSETZUNGEN

COMPOSITIONS TOPIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.01.2018 US 201862623309 P**
**12.10.2018 US 201862745078 P**

(43) Date of publication of application:
**09.12.2020 Bulletin 2020/50**

(73) Proprietor: **Mary Kay, Inc.**
**Addison, TX 75001 (US)**

(72) Inventors:
• **NORMAN, Greg**
**Bedford, Texas 76021 (US)**
• **SANCHEZ, Milagros**
**Dallas Parkway Addison, Texas 75001 (US)**
• **CUMMINGS, Kathlene**
**Dallas Parkway Addison, Texas 75001 (US)**
• **KALAHASTI, Geetha**
**Plano, Texas 75024 (US)**
• **ZHAO, Wanli**
**Dallas Parkway Addison, Texas 75001 (US)**
• **VANPELT, Lisha**
**Dallas Parkway Addison, Texas 75001 (US)**
• **JACOBY, Patricia**
**Dallas Parkway Addison, Texas 75001 (US)**
• **GAN, David**
**Southlake, Texas 76092 (US)**
• **FRUSHOUR, Michael**
**Dallas, Texas 75001 (US)**

(74) Representative: **Uexküll & Stolberg**
**Partnerschaft von**
**Patent- und Rechtsanwälten mbB**
**Beselerstraße 4**
**22607 Hamburg (DE)**

(56) References cited:
**EP-A2- 3 897 867**    **CN-A- 107 412 042**
**KR-A- 20160 068 310**    **US-A1- 2007 134 193**
**US-A1- 2017 020 808**    **US-A1- 2017 027 856**
**US-A1- 2019 038 689**

## Description

The present application claims priority to U.S. provisional patent applications 62/623,309 filed January 29, 2018, and 62/745,078 filed October 12, 2018.

## BACKGROUND OF THE INVENTION

### A. Field of the Invention

The present invention relates generally to topical compositions that can be used to improve skin conditions and/or block pollutants from the skin. In certain aspects, the topical compositions are capable of inhibiting nitric oxide synthase, decreasing TNF-$\alpha$ production, increasing the production of barrier proteins, and/or blocking the accumulation of PM 2.5 particles. The combination of ingredients includes *Myrothamnus flabellifolia* extract and *Alteromonas* ferment extract, and, optionally, saccharide isomerate, and/or *Opuntia tuna* fruit extract.

### B. Description of Related Art

Aging, chronic exposure to adverse environmental factors, malnutrition, fatigue, etc., can change the visual appearance, physical properties, or physiological functions of skin in ways that are considered visually undesirable. The most notable and obvious changes include the development of fine lines and wrinkles, loss of elasticity, increased sagging, loss of firmness, loss of color evenness or tone, coarse surface texture, and mottled pigmentation. Less obvious but measurable changes which occur as skin ages or endures chronic insult include a general reduction in cellular and tissue vitality, reduction in cell replication rates, reduced cutaneous blood flow, reduced moisture content, accumulated errors in structure and function, alterations in the normal regulation of common biochemical pathways, and a reduction in the skin's ability to remodel and repair itself. Many of the alterations in appearance and function of the skin are caused by changes in the outer epidermal layer of the skin, while others are caused by changes in the lower dermis. For instance, barrier proteins, such as Filaggrin and Occludin-1, are located in the outer layers of the epidermis and are essential to skin function and appearance. Filaggrin is the precursor to Natural Moisturizing Factor (NMF) in the skin. Increased NMF increases the moisture content of the skin. Occludin-1 is a critical protein to the formulation of tight junctions and the skin's moisture barrier function. Thus, a change of production rate for these barrier proteins can effectively alter the appearance and the conditions of the skin.

Many factors contribute to skin aging and associated alterations in appearance and function of the skin, such as the actual age of a person, the amount of exposure to environmental factors (e.g., sun light, pollution, chemicals, smoke, etc.), and how well a person has taken care of their skin. In particular, skin aging concerns two processes - intrinsic aging, which is related to the natural aging process and genetic influences, and extrinsic aging, which is accumulated damage due to environmental factors.

Intrinsic aging process in cells and skin can be related to the loss of proper function of the skin in maintaining biochemical pathways. Such pathways can control the oxidative/reductive environment balance in the skin, the regulation of inflammation, and the maintenance of the moisture balance of the skin. Losses of proper function of the skin can lead to increased oxidative damage, increased inflammation, dry skin, loss of skin firmness, increased skin unevenness, and increased fine lines and wrinkles.

Factors that cause extrinsic aging can include exposure to ultraviolet (UV) rays, irritants, and pollutants, such as fine particles suspended in the air. UV rays, through sun exposure or the use of ultraviolet lamps (for example, tanning beds), can induce oxidative stress, inflammation, production of melanin, and even genetic mutations that leads to skin damage. The accumulation of oxidative stress through free radical formation can damage skin proteins leading to skin aging, which includes loss of elasticity, loss of dermal proteins, lines and wrinkles, and abnormal pigmentation. Similarly, accumulation of fine particles such as PM 2.5 (fine particles that have an aerodynamic diameter under 2.5 $\mu$m) on the skin can also induce oxidative stress and inflammation. These fine particles may also cause damage on barrier proteins of the skin, leading to loss of moisture and elasticity of the skin.

The combination of intrinsic and extrinsic factors eventually leads to visible alterations of the appearance and function of the skin. Current products on the market either do not effectively address the signs or causes of aging and/or alternations of appearance and function of the skin. Moreover, current products often fail to address the effects of extrinsic factors on the skin and/or they have skin irritating effects. For example, current products may not address loss of skin firmness, pigmentation problems, appearance of fine lines or wrinkles, and/or loss of moisture that are caused by fine particles or chemical pollutions in the atmosphere.

US 2017/020808 A1 relates to compositions useful for application to skin and hair comprising saccharide isomerate, *Alteromonas* ferment extract, and a dermatologically acceptable vehicle. KR 2016 0068310 A relates to topical cosmetic compositions comprising *Myrothamnus flabellifolia* extract.

## SUMMARY OF THE INVENTION

The inventors have determined a solution to at least some of the problems associated with current products to counteract some of the intrinsic and/or extrinsic factors that change the appearance and/or condition of skin and eventually cause skin aging. The solution resides in a combination of ingredients including *Myrothamnusflabellifolia* extract *and Alteromonas* ferment extract, and, optionally, saccharide isomerate and/or *Opuntia tuna* fruit extract. The combination of ingredients can be used to create a topical skin composition to improve overall skin appearance, improve moisture content, improve texture/smoothness, improve firmness, counter oxidative damage, reduce oxidizing agents, increase the oxidative capacity of a composition, increase production of dermal proteins (e.g., Filaggrin and Occludin-1), inhibit nitric oxidative synthase, inhibit TNF-$\alpha$, detoxify heavy metals (e.g., cadmium and lead) by chelation, and/or block accumulation of fine particulate matter on the skin.

In some aspects, there is disclosed a topical composition as defined in the claims. The topical composition includes (a) an effective amount of *Myrothamnus flabellifolia* extractand *Alteromonas* ferment extract. In some instances, the composition further includes an effective amount of saccharide isomerate and/or *Opuntia tuna* fruit extract. The amounts of these ingredients within the composition can vary. For example, the amounts of each ingredient can be as low as 0.000001% to as high as 98% w/w or any range therein. In one aspect, the amount of each of the aforementioned ingredients (*Myrothamnus flabellifolia* extract, saccharide isomerate, *Alteromonas* ferment extract, and *Opuntia tuna* fruit extract) in a composition can be 0.0001 wt. % to 3 wt. % or 0.0001 wt. % to 2 wt. %, or 0.0001 wt. % to 1 wt. %. In some aspects, the topical composition can include 0.0001 to 1% by weight of *Myrothamnus flabellifolia* extract and all ranges and values there between including 0.0001 to 0.005%, 0.005 to 0.01%, 0.01 to 0.05%, 0.05 to 0.1%, 0.1 to 0.5%, and 0.5 to 1%. The topical composition can include 0.0001 to 1% by weight of saccharide isomerate and all ranges and values there between including 0.0001 to 0.001%, 0.001 to 0.01%, 0.01 to 0.05%, 0.05 to 0.1%, 0.1 to 0.5%, and 0.5 to 1%. The topical composition can include 0.001 to 1% by weight of *Alteromonas* ferment and all ranges and values there between, including 0.001 to 0.01%, 0.01 to 0.05%, 0.05 to 0.1%, 0.1 to 0.5%, and 0.5 to 1%. In some embodiments, the composition comprises 0.001 to 0.03% by weight of *Myrothamnus flabellifolia* extract, 0.0001 to 0.02% by weight of saccharide isomerate, 0.001 to 0.03% by weight of *Alteromonas* ferment extract, or combinations thereof. In some aspects, the composition comprises 0.0001 to 1% by weight of *Myrothamnus flabellifolia* extract, 0.0001 to 1% by weight of saccharide isomerate, 0.001 to 1% by weight of *Alteromonas* ferment extract, or combinations thereof. Optionally, the topical composition can include 0.00001 to 3% by weight of *Opuntia tuna* fruit extract and all ranges and values there between including 0.00001 to 0.0001%, 0.0001 to 0.001%, 0.001 to 0.01%, 0.01 to 0.1%, 0.1 to 0.3%, 0.3 to 0.6%, 0.6 to 0.9%, 0.9 to 1.2%, 1.2 to 1.5%, 1.5 to 1.8%, 1.8 to 2.1%, 2.1 to 2.4%, 2.4 to 2.7%, and 2.7 to 3.0%.

In some aspects, the topical composition includes an effective amount of *Myrothamnusflabellifolia* extract and/or saccharide isomerate to increase production of barrier proteins of skin. In some aspects, the topical composition can include an effective amount of *Myrothamnus flabellifolia* extract and/or saccharide isomerate to block accumulation of PM 2.5 particles on skin. In some aspects, the topical composition can include an effective amount of *Myrothamnus flabellifolia* extract and/or saccharide isomerate to inhibit nitric oxide synthase (NOS) and TNF-$\alpha$.

In some instances, the extracts can be aqueous extracts. By aqueous extracts, it is meant that an aqueous solution can be used as the extractant or solvent to obtain the extract. The aqueous extracts can be in liquid form or in powdered form. In some instances, other solvents such as alcohols, glycols, hydro-alcoholic, and/or hydroglycolic extracts can be used. In some instances, the composition further comprises water. In some instances, the composition includes 25% to 98% by weight of water.

In some aspects, the *Myrothamnusflabellifolia* extract can be an aqueous extract of stems and/or leaves of *Myrothamnus flabellifolia*. The saccharide isomerate can include an exopolysaccharide of *Vibrio alginolyticus* belonging to the family of Thalasso plankton and can be an aqueous extract. The *Alteromonas* ferment extract can include an exopolysaccharide from Kopara and can be an aqueous-alcoholic extract. The Kopara can be from the rim of a French Polynesian atoll.

In some aspects, the topical compositions may further include sunscreen agent. The compositions can be sunscreen lotions, sprays, or creams. Non-limiting examples for the sunscreen agents may include, but are not limited to, octinoxate, zinc oxide, ethylhexyl salicylate, ensulizole, homosalate, avobenzone, octocrylene, oxybenzone, or combinations thereof. In some instances, the topical composition may have a sun protection factor (SPF) in a range of 2 to 100 and all ranges and values there between such as SPF 2, SPF 15, SPF 25, SPF 30, SPF 35, SPF 40, SPF 50, SPF 60, SPF 70, SPF 75, SPF 80, SPF 90 and SPF 100.

The topical compositions of the present invention can also include any one of, any combination of, or all of the following additional ingredients: water, a chelating agent, a moisturizing agent, a preservative, a thickening agent, a silicone containing compound, an essential oil, a structuring agent, a vitamin, a pharmaceutical ingredient, or an anti-oxidant, or any combination of such ingredients or mixtures of such ingredients. In some aspects, the composition can include at least two, three, four, five, six, seven, eight, nine, ten, or all of these additional ingredients identified in the previous sentence. Examples of these additional ingredients are identified throughout this specification. The amounts of such ingredients can range from 0.0001% to 99.9% by weight or volume of the composition, or any integer or range in between as disclosed in other sections of this specification. The topical composition can be formulated as a mask, lotion, cleanser, moisturizer, cream, gel, eye gel, serum, emulsion (e.g., oil-in-water, water-in-oil, silicone-in-water, water-in-silicone, water-in-oil-in-water, oil-in-water-in-oil, oil-in-water-in-silicone, etc.), gel emulsion, gel serum, solutions (e.g., aqueous or hydro-alcoholic solutions), anhydrous bases (e.g., lipstick or a powder), ointments, milk, paste, aerosol, solid forms, eye jellies, gel serums, gel emulsions, etc. The topical composition can be formulated for topical skin application at least 1, 2, 3, 4, 5, 6, 7, or more times a day during use. In other aspects of the present invention, compositions can be storage stable or color stable, or both. It is also contemplated that the viscosity of the composition can be selected to achieve a desired result, e.g., depending on the type of composition desired, the viscosity of such composition can be from about 1 cps to well over 1 million cps or any range or integer derivable therein (e.g., 2 cps, 3, 4, 5, 6, 7, 8, 9, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, 600000, 700000, 800000, 900000, 1000000, 2000000, 3000000, 4000000, 5000000, 10000000, cps, etc., as measured on a Brookfield Viscometer using a TC spindle at 2.5 rpm at 25°C).

The compositions can have a pH of about 6 to about 9. In other aspects, the pH can be 1, 2, 3, 4, 5, 6, 7, 8, 9, 10, 11, 12, 13, or 14. The compositions can include a triglyceride. Non-limiting examples include small, medium, and large chain triglycerides. In certain aspects, the triglyceride is a medium chain triglyceride (e.g., caprylic capric triglyceride). The compositions can also include preservatives. Non-limiting examples of preservatives include methylparaben, propylparaben, or a mixture of methylparaben and propylparaben. In some embodiments, the composition is paraben-free.

Kits that include the compositions of the present invention are also contemplated. In some embodiments, the composition is comprised in a container. The container can be a bottle, dispenser, or package. The container can dispense a pre-determined amount of the composition. In some aspects, the compositions is dispensed in a spray, mist, dollop, or liquid. The container can include indicia on its surface. The indicia can be a word, an abbreviation, a picture, or a symbol.

It is also contemplated that the compositions disclosed throughout this specification can be used as a leave-on or rinse-off composition. By way of example, a leave-on composition can be one that is topically applied to skin and remains on the skin for a period of time (e.g., at least 5, 6, 7, 8, 9, 10, 20, or 30 minutes, or at least 1, 2, 3, 4, 5, 6, 7, 8, 9, 10, 11, 12, 13, 14, 15, 16, 17, 18, 19, 20, 21, 22, 23 or 24 hours, or overnight or throughout the day). Alternatively, a rinse-off composition can be a product that is intended to be applied to the skin and then removed or rinsed from the skin (e.g., with water) within a period of time such as less than 5, 4, 3, 2, or 1 minute. An example of a rinse off composition can be a skin cleanser, shampoo, conditioner, or soap. An example of a leave-on composition can be a skin moisturizer, sunscreen, mask, overnight cream, or a day cream.

In embodiments of the invention, there is disclosed a method of improving a condition or appearance of skin. The method include applying a topical composition comprising (a) an effective amount of *Myrothamnus flabellifolia* extract *and Alteromonas* ferment extract, and, optionally, (b) an effective amount of saccharide isomerate and/or *Opuntia tuna* fruit extract as defined in the claims to skin in need thereof. In certain aspects, the condition or appearance of skin to be improved can include a red blotch on skin, a dark circle on, under, or around an eye, skin tone, skin firmness, moisture content of the skin, accumulation of fine particles on the skin, and/or overall skin appearance.

In certain aspects, the skin is treated to reduce the nitric oxide synthase, reduce TNF-$\alpha$, increase barrier proteins, and/or block the accumulation of fine particles thereon. In certain aspects, the barrier proteins can include Occludin-1, Filaggrin and/or other barrier proteins known in the art. In some instances, non-limiting examples for fine particles can include particles with an aerodynamic diameter equal to or less than 2.5 $\mu$m (PM 2.5). In some aspects, the *Myrothamnus flabellifolia* extract is capable of inhibiting Nitric Oxide Synthase, inhibiting TNF-$\alpha$, and blocking accumulation of PM 2.5. In some aspects, the saccharide isomerate is capable of inhibiting Nitric Oxide Synthase, inhibiting TNF-$\alpha$, and increasing production rate of Occludin-1 and Filaggrin barrier proteins. In some aspects, the *Alteromonas* ferment extract is capable of increasing production of hyaluronic acid, inhibiting hyaluronidase, inhibiting elastase, and detoxifying heavy metals (e.g., cadmium and lead) by chelation.

In some aspects, the *Myrothamnus flabellifolia* extract can be an aqueous extract of stems and/or leaves of *Myrothamnus flabellifolia.* The saccharide isomerate can contain an exopolysaccharide of *Vibrio alginolyticus* belonging to the family of Thalasso plankton. The *Alteromonas* ferment extract can include an exopolysaccharide from Kopara. In some aspects, the composition in the method can include 0.001 to 0.03% by weight of *Myrothamnus flabellifolia* extract, 0.0001 to 0.02% by weight of saccharide isomerate, and 0.001 to 0.03% by weight of *Alteromonas* ferment extract.

It is contemplated that any aspect or embodiment discussed in this specification can be implemented with respect to any method or composition of the invention, and *vice versa.* Furthermore, compositions of the invention can be used to achieve methods of the invention.

In one embodiment, compositions of the present invention can be pharmaceutically or cosmetically elegant or can have pleasant tactile properties. "Pharmaceutically elegant," "cosmetically elegant," and/or "pleasant tactile properties" describes a composition that has particular tactile properties which feel pleasant on the skin (e.g., compositions that are not too watery or greasy, compositions that have a silky texture, compositions that are non-tacky or sticky, etc.). Pharmaceutically or cosmetically elegant can also relate to the creaminess or lubricity properties of the composition or to the moisture retaining properties of the composition.

Also contemplated is a product comprising a composition of the present invention. In non-limiting aspects, the product can be a cosmetic product. The cosmetic product can be those described in other sections of this specification or those known to a person of skill in the art. Non-limiting examples of products include a moisturizer, a cream, a lotion, a skin softener, a gel, a wash, a foundation, a night cream, a lipstick, a cleanser, a toner, a sunscreen, a mask, an anti-aging product, a deodorant, an antiperspirant, a perfume, a cologne, etc.

"Topical application" means to apply or spread a composition onto the surface of lips or keratinous tissue. "Topical skin composition" includes compositions suitable for topical application on skin and/or keratinous tissue. Such compositions are typically dermatologically-acceptable in that they do not have undue toxicity, incompatibility, instability, allergic response, and the like, when applied to skin and/or keratinous tissue. Topical skin care compositions of the present invention can have a selected viscosity to avoid significant dripping or pooling after application to skin and/or keratinous tissue.

"Keratinous tissue" includes keratin-containing layers disposed as the outermost protective covering of mammals and includes, but is not limited to, lips, skin, hair, and nails.

The term "about" or "approximately" are defined as being close to as understood by one of ordinary skill in the art. In one non-limiting embodiment the terms are defined to be within 10%, preferably within 5%, more preferably within 1%, and most preferably within 0.5%.

The term "substantially" and its variations are refers to ranges within 10%, within 5%, within 1%, or within 0.5%.

The terms "wt.%", "vol.%", or "mol.%" refers to a weight, volume, or molar percentage of a component (e.g., ingredient), respectively, based on the total weight, the total volume, or the total moles of material (e.g., topical composition) that includes the component. In a non-limiting example, 10 grams of component in 100 grams of the material is 10 wt. % of component.

The terms "inhibiting" or "reducing" or any variation of these terms includes any measurable decrease or complete inhibition to achieve a desired result. The terms "promote" or "increase" or any variation of these terms includes any measurable increase or production of a protein or molecule (e.g., matrix proteins such as fibronectin, laminin, collagen, or elastin or molecules such as hyaluronic acid) to achieve a desired result.

The term "effective," as that term is used in the specification and/or claims, means adequate to accomplish a desired, expected, or intended result.

The use of the word "a" or "an" when used in conjunction with the terms "comprising," "including," "having," or "containing," or any variations of these terms, in the claims and/or the specification may mean "one," but it is also consistent with the meaning of "one or more," "at least one," and "one or more than one."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The compositions and methods for their use can "comprise," "consist essentially of," or "consist of" any of the ingredients or steps disclosed throughout the specification. With respect to the phrase "consisting essentially of," a basic and novel property of the compositions and methods of the present invention is their ability to reduce skin inflammation, treat skin aging, and/or reduce accumulation of fine particles on the skin.

Other objects, features, and advantages of the present invention will become apparent from the following detailed description. It should be understood, however, that the detailed description and the examples, while indicating specific embodiments of the invention, are given by way of illustration only.

## BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

FIG. 1 shows microscopic images of treated and non-treated human keratinocytes by *Myrothamnus flabellifolia* extract after exposure to PM 2.5 for 24 hours.

FIG. 2 shows an example of four (4) testing areas on a human subject face to evaluate the efficacy of a combination of an embodiment of the cleanser and freshener to remove fine particulate matter and the efficacy of an embodiment of the freshener to remove excess sebum.

EP 3 746 043 B1

FIG. 3A shows the biosorption curve of cadmium retention by *Alteromonas* ferment extract at isotherm using $Cd(NO_3)_2$ as the salt.

FIG. 3B is the linear transformation of the Langmuir equation that shows the quantification of cadmium chelation by *Alteromonas* ferment extract at isotherm using $Cd(NO_3)_2$ as the salt.

FIG. 4A shows the biosorption curve of lead retention by *Alteromonas* ferment extract at isotherm using $Pb(NO_3)_2$ as the salt.

FIG. 4B is the linear transformation of the Langmuir equation that shows the quantification of lead chelation by *Alteromonas* ferment extract at isotherm using $Pb(NO_3)_2$ as the salt.

## DESCRIPTION OF ILLUSTRATIVE EMBODIMENTS

As noted above, the present invention provides a solution to at least some of the problems associated with skin inflammation, skin aging, and accumulation of fine particles on the skin. The solution is premised on combinations of extracts and other compounds as defined in the claims to inhibit nitric oxide synthase, inhibit TNF-$\alpha$, increase barrier protein production rate, and/or blocking accumulation of fine particles on the barrier proteins of the skin. The combinations of extracts and compounds include (a) an effective amount of *Myrothamnus flabellifolia* extract and *Alteromonas* ferment extract; and, optionally, (b) an effective amount of saccharide isomerate and/or *Opuntia tuna* fruit extract. As illustrated in a non-limiting manner in the Examples, this combination has been shown to reduce nitric oxide synthase activity, decrease TNF-$\alpha$ production, increase the production of Occludin-1 and/or Filaggrin in human skin cells such as human epidermal keratinocytes, increase production of hyaluronic acid, inhibit hyaluronidase, detoxify heavy metals (e.g., cadmium and lead) by chelation, inhibit elastase, and block accumulation of PM 2.5 particles on skin.

These and other aspects of the present invention are described in the following sections.

## A. Active Ingredients

The present invention is premised on a determination that a combination of active ingredients- (a) an effective amount of *Myrothamnus flabellifolia* extract and *Alteromonas* ferment extract; and, optionally, (b) an effective amount of saccharide isomerate and/or Opuntia tuna fruit extract-can be used to improve the skin's visual appearance, reduce skin inflammation, and block fine particle accumulation on the skin. More particularly, the combination can be used to reduce nitric oxide synthase activity, decrease TNF-$\alpha$ production, and increase the production of Occludin-1 and/or Filaggrin in human skin cells such as human epidermal keratinocytes, detoxify heavy metals (e.g., cadmium and lead) by chelation, and block accumulation of PM 2.5 particles on skin cells such as human keratinocytes.

This combination of ingredients can be used in different products to treat various skin conditions. By way of non-limiting examples, the combination of ingredients can be formulated in an emulsion (e.g., oil-in-water, water-in-oil), a gel, a serum, a gel emulsion, a gel serum, a lotion, a mask, or a body butter.

*Myrothamnus flabellifolia* extract is an extract of *Myrothamnus flabellifolia,* also known as the resurrection plant, a flowering plant native to Southern Africa. In some instances, *Myrothamnusflabellifolia* extract is commercially available. In some instances, *Myrothamnus flabellifolia* extract can be supplied by Rahn under the trade name MYRAMAZE®. In some instances, the extract can be an aqueous extract or an alcohol extract. In some instances, the extract is a water extract. In some instances, the extract is an extract of the whole plant or one or more parts of the plant. In some instances, the extract is an extract of the leaf and stem of the plant.

Saccharide isomerate is an exopolysaccharide synthesized by a micro-organism called *Vibrio alginolyticus* and belonging to the family of Thalasso plankton. In some instances, saccharide isomerate is commercially available. In some instances, saccharide isomerate can be supplied by Barnet Products under the trade name BENOIDERM®. In some instances, the extract can be an aqueous extract or an alcohol extract. In some instances, the extract can be an aqueous extract.

*Alteromonas* ferment extract is exopolysaccharides from "Kopara" (microorganisms mat) living in a unique ecosystem in the rims of French Polynesian atolls. In some embodiments this ingredient is commercially available, e.g., from Lucas Meyer under the trade name Exo-H™ and EXO-P™. The exopolysaccharide can have a molecular weight range of between 100 kDa and 5,000 kDa, preferably a molecular weight range of between 500 kDa and 3,000 kDa, and more preferably a molecular weight range of between 800 kDa and 1,500 kDa. In some instances, the mean average molecular weight of the exopolysaccharide is 1,000 kDa. In some instances, the extract is an aqueous extract. In some instances, the extract is an aqueous-alcoholic extract. It has been determined that this ingredient can be used to increase production of hyaluronic acid, inhibit hyaluronidase, inhibit elastase, and detoxify skin by chelating heavy metals (e.g., cadmium and lead).

*Opuntia tuna* fruit extract is an extract from the fruit of prickly pear. The extract is commercially available and can be obtained from a variety of commercial sources (see International Cosmetic Ingredient Dictionary and Handbook, 12th edition, volume 2, page 1731 (2008)).

The extracts described herein can be extracts made through extraction methods known in the art and combinations thereof. Non-limiting examples of extraction methods include the use of liquid-liquid extraction, solid phase extraction, aqueous extraction, ethyl acetate, alcohol, acetone, oil, supercritical carbon dioxide, heat, pressure, pressure drop extraction, ultrasonic extraction, etc. Extracts can be a liquid, solid, dried liquid, re-suspended solid, etc.

**B. Amounts of Ingredients**

It is contemplated that the compositions of the present invention can include any amount of the ingredients discussed in this specification. The compositions can also include any number of combinations of additional ingredients described throughout this specification (e.g., pigments, or additional cosmetic or pharmaceutical ingredients). The concentrations of any ingredient within the compositions can vary. In non-limiting embodiments, for example, the compositions can comprise, consisting essentially of, or consist of, in their final form, for example, at least about 0.0001%, 0.0002%, 0.0003%, 0.0004%, 0.0005%, 0.0006%, 0.0007%, 0.0008%, 0.0009%, 0.0010%, 0.0011%, 0.0012%, 0.0013%, 0.0014%, 0.0015%, 0.0016%, 0.0017%, 0.0018%, 0.0019%, 0.0020%, 0.0021%, 0.0022%, 0.0023%, 0.0024%, 0.0025%, 0.0026%, 0.0027%, 0.0028%, 0.0029%, 0.0030%, 0.0031%, 0.0032%, 0.0033%, 0.0034%, 0.0035%, 0.0036%, 0.0037%, 0.0038%, 0.0039%, 0.0040%, 0.0041%, 0.0042%, 0.0043%, 0.0044%, 0.0045%, 0.0046%, 0.0047%, 0.0048%, 0.0049%, 0.0050%, 0.0051%, 0.0052%, 0.0053%, 0.0054%, 0.0055%, 0.0056%, 0.0057%, 0.0058%, 0.0059%, 0.0060%, 0.0061%, 0.0062%, 0.0063%, 0.0064%, 0.0065%, 0.0066%, 0.0067%, 0.0068%, 0.0069%, 0.0070%, 0.0071%, 0.0072%, 0.0073%, 0.0074%, 0.0075%, 0.0076%, 0.0077%, 0.0078%, 0.0079%, 0.0080%, 0.0081%, 0.0082%, 0.0083%, 0.0084%, 0.0085%, 0.0086%, 0.0087%, 0.0088%, 0.0089%, 0.0090%, 0.0091%, 0.0092%, 0.0093%, 0.0094%, 0.0095%, 0.0096%, 0.0097%, 0.0098%, 0.0099%, 0.0100%, 0.0200%, 0.0250%, 0.0275%, 0.0300%, 0.0325%, 0.0350%, 0.0375%, 0.0400%, 0.0425%, 0.0450%, 0.0475%, 0.0500%, 0.0525%, 0.0550%, 0.0575%, 0.0600%, 0.0625%, 0.0650%, 0.0675%, 0.0700%, 0.0725%, 0.0750%, 0.0775%, 0.0800%, 0.0825%, 0.0850%, 0.0875%, 0.0900%, 0.0925%, 0.0950%, 0.0975%, 0.1000%, 0.1250%, 0.1500%, 0.1750%, 0.2000%, 0.2250%, 0.2500%, 0.2750%, 0.3000%, 0.3250%, 0.3500%, 0.3750%, 0.4000%, 0.4250%, 0.4500%, 0.4750%, 0.5000%, 0.5250%, 0.0550%, 0.5750%, 0.6000%, 0.6250%, 0.6500%, 0.6750%, 0.7000%, 0.7250%, 0.7500%, 0.7750%, 0.8000%, 0.8250%, 0.8500%, 0.8750%, 0.9000%, 0.9250%, 0.9500%, 0.9750%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 5.1%, 5.2%, 5.3%, 5.4%, 5.5%, 5.6%, 5.7%, 5.8%, 5.9%, 6.0%, 6.1%, 6.2%, 6.3%, 6.4%, 6.5%, 6.6%, 6.7%, 6.8%, 6.9%, 7.0%, 7.1%, 7.2%, 7.3%, 7.4%, 7.5%, 7.6%, 7.7%, 7.8%, 7.9%, 8.0%, 8.1%, 8.2%, 8.3%, 8.4%, 8.5%, 8.6%, 8.7%, 8.8%, 8.9%, 9.0%, 9.1%, 9.2%, 9.3%, 9.4%, 9.5%, 9.6%, 9.7%, 9.8%, 9.9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 60%, 65%, 70%, 75%, 80%, 85%, 90%, 95%, or 99% or any range derivable therein, of at least one of the ingredients that are mentioned throughout the specification and claims. In non-limiting aspects, the percentage can be calculated by weight or volume of the total composition. A person of ordinary skill in the art would understand that the concentrations can vary depending on the addition, substitution, and/or subtraction of ingredients in a given composition.

**C. Vehicles**

The compositions of the present invention can include or be incorporated into all types of vehicles and carriers. The vehicle or carrier can be a pharmaceutically or dermatologically acceptable vehicle or carrier. Non-limiting examples of vehicles or carriers include water, glycerin, alcohol, oil, a silicon containing compound, a silicone compound, and wax. Variations and other appropriate vehicles will be apparent to the skilled artisan and are appropriate for use in the present invention. In certain aspects, the concentrations and combinations of the compounds, ingredients, and agents can be selected in such a way that the combinations are chemically compatible and do not form complexes which precipitate from the finished product.

**D. Structure**

The compositions of the present invention can be structured or formulated into a variety of different forms. Non-limiting examples include emulsions (e.g., water-in-oil, water-in-oil-in-water, oil-in-water, silicone-in-water, water-in-silicone, oil-in-water-in-oil, oil-in-water-in-silicone emulsions), creams, lotions, solutions (both aqueous and hydro-alcoholic), anhydrous bases (such as lipsticks and powders), gels, masks, peels, and ointments. Variations and other structures will be apparent to the skilled artisan and are appropriate for use in the present invention.

### E. Additional Ingredients

In addition to the combination of ingredients disclosed by the inventors, the compositions can also include additional ingredients such as cosmetic ingredients and pharmaceutical active ingredients. Non-limiting examples of these additional ingredients are described in the following subsections.

### 1. Cosmetic Ingredients

The CTFA International Cosmetic Ingredient Dictionary and Handbook (2004 and 2008) describes a wide variety of non-limiting cosmetic ingredients that can be used in the context of the present invention. Examples of these ingredient classes include: fragrance agents (artificial and natural; e.g., gluconic acid, phenoxyethanol, and triethanolamine), dyes and color ingredients (e.g., Blue 1, Blue 1 Lake, Red 40, titanium dioxide, D&C blue no. 4, D&C green no. 5, D&C orange no. 4, D&C red no. 17, D&C red no. 33, D&C violet no. 2, D&C yellow no. 10, and D&C yellow no. 11), flavoring agents / aroma agents (e.g., *Stevia rebaudiana* (sweetleaf) extract, and menthol), adsorbents, lubricants, solvents, moisturizers (including, e.g., emollients, humectants, film formers, occlusive agents, and agents that affect the natural moisturization mechanisms of the skin), water-repellants, UV absorbers and/or reflectors (physical and chemical absorbers such as para-aminobenzoic acid ("PABA") and corresponding PABA derivatives, titanium dioxide, zinc oxide, etc.), essential oils, vitamins (e.g., A, B, C, D, E, and K), trace metals (e.g., zinc, calcium and selenium), anti-irritants (e.g., steroids and non-steroidal anti-inflammatories), botanical extracts (e.g., *Aloe vera,* chamomile, cucumber extract, *Ginkgo biloba,* ginseng, and rosemary), anti-microbial agents, antioxidants (e.g., BHT and tocopherol), chelating agents (e.g., disodium EDTA and tetrasodium EDTA), preservatives (e.g., methylparaben and propylparaben), pH adjusters (e.g., sodium hydroxide and citric acid), absorbents (e.g., aluminum starch octenylsuccinate, kaolin, corn starch, oat starch, cyclodextrin, talc, and zeolite), skin bleaching and lightening agents (e.g., hydroquinone and niacinamide lactate), humectants (e.g., sorbitol, urea, methyl gluceth-20, saccharide isomerate, and mannitol), exfoliants, waterproofing agents (e.g., magnesium/aluminum hydroxide stearate), skin conditioning agents (e.g., aloe extracts, allantoin, bisabolol, ceramides, dimethicone, hyaluronic acid, biosaccharide gum-1, ethylhexylglycerin, pentylene glycol, hydrogenated polydecene, octyldodecyl oleate, and dipotassium glycyrrhizate). Non-limiting examples of some of these ingredients are provided in the following subsections.

### a. UV Absorption and/or Reflecting Agents

UV absorption and/or reflecting agents that can be used in combination with the compositions of the present invention include chemical and physical sunblocks. Non-limiting examples of chemical sunblocks that can be used include para-aminobenzoic acid (PABA), PABA esters (glyceryl PABA, amyldimethyl PABA and octyldimethyl PABA), butyl PABA, ethyl PABA, ethyl dihydroxypropyl PABA, benzophenones (oxybenzone, sulisobenzone, benzophenone, and benzophenone-1 through 12), cinnamates (octyl methoxycinnamate, isoamyl p-methoxycinnamate, octylmethoxy cinnamate, cinoxate, diisopropyl methyl cinnamate, DEA-methoxycinnamate, ethyl diisopropylcinnamate, glyceryl octanoate dimethoxycinnamate and ethyl methoxycinnamate), cinnamate esters, salicylates (homomethyl salicylate, benzyl salicylate, glycol salicylate, isopropylbenzyl salicylate, etc.), anthranilates, ethyl urocanate, homosalate, octisalate, dibenzoylmethane derivatives (e.g., avobenzone), octocrylene, octyl triazone, digalloyl trioleate, glyceryl aminobenzoate, lawsone with dihydroxyacetone, ethylhexyl triazone, dioctyl butamido triazone, benzylidene malonate polysiloxane, terephthalylidene dicamphor sulfonic acid, disodium phenyl dibenzimidazole tetrasulfonate, diethylamino hydroxybenzoyl hexyl benzoate, bis diethylamino hydroxybenzoyl benzoate, bis benzoxazoylphenyl ethylhexylimino triazine, drometrizole trisiloxane, methylene bis-benzotriazolyl tetramethylbutylphenol, and bis-ethylhexyloxyphenol methoxyphenyltriazine, 4-methylbenzylidene camphor, and isopentyl 4-methoxycinnamate. Non-limiting examples of physical sunblocks include, kaolin, talc, petrolatum and metal oxides (e.g., titanium dioxide and zinc oxide).

### b. Moisturizing Agents

Non-limiting examples of moisturizing agents that can be used with the compositions of the present invention include amino acids, chondroitin sulfate, diglycerin, erythritol, fructose, glucose, glycerin, glycerol polymers, glycol, 1,2,6-hexanetriol, honey, hyaluronic acid, hydrogenated honey, hydrogenated starch hydrolysate, inositol, lactitol, maltitol, maltose, mannitol, natural moisturizing factor, PEG-15 butanediol, polyglyceryl sorbitol, saccharide isomerate, salts of pyrrolidone carboxylic acid, potassium PCA, propylene glycol, sodium glucuronate, sodium PCA, sorbitol, sucrose, trehalose, urea, and xylitol.

Other examples include acetylated lanolin, acetylated lanolin alcohol, alanine, algae extract, *Aloe barbadensis,*

*Aloe barbadensis* extract, *Aloe barbadensis* gel, *Althea officinalis* extract, apricot (*Prunus armeniaca*) kernel oil, arginine, arginine aspartate, *Arnica montana* extract, aspartic acid, avocado (*Persea gratissima*) oil, barrier sphingolipids, butyl alcohol, beeswax, behenyl alcohol, beta-sitosterol, birch (*Betula alba*) bark extract, borage (*Borago officinalis*) extract, butcherbroom (*Ruscus aculeatus*) extract, butylene glycol, *Calendula officinalis* extract, *Calendula officinalis* oil, candelilla (*Euphorbia cerifera*) wax, canola oil, caprylic/capric triglyceride, cardamom (*Elettaria cardamomum*) oil, carnauba (*Copernicia cerifera*) wax, carrot (*Daucus carota sativa*) oil, castor (*Ricinus communis*) oil, ceramides, ceresin, ceteareth-5, ceteareth-12, ceteareth-20, cetearyl octanoate, ceteth-20, ceteth-24, cetyl acetate, cetyl octanoate, cetyl palmitate, chamomile (*Anthemis nobilis*) oil, cholesterol, cholesterol esters, cholesteryl hydroxystearate, citric acid, clary (*Salvia sclarea*) oil, cocoa (*Theobroma cacao*) butter, coco-caprylate/caprate, coconut (*Cocos nucifera*) oil, collagen, collagen amino acids, corn (*Zea mays*) oil, fatty acids, decyl oleate, dimethicone copolyol, dimethiconol, dioctyl adipate, dioctyl succinate, dipentaerythrityl hexacaprylate/hexacaprate, DNA, erythritol, ethoxydiglycol, ethyl linoleate, *Eucalyptus globulus* oil, evening primrose (*Oenothera biennis*) oil, fatty acids, *Geranium maculatum* oil, glucosamine, glucose glutamate, glutamic acid, glycereth-26, glycerin, glycerol, glyceryl distearate, glyceryl hydroxystearate, glyceryl laurate, glyceryl linoleate, glyceryl myristate, glyceryl oleate, glyceryl stearate, glyceryl stearate SE, glycine, glycol stearate, glycol stearate SE, glycosaminoglycans, grape (*Vitis vinifera*) seed oil, hazel (*Corylus americana*) nut oil, hazel (*Corylus avellana*) nut oil, hexylene glycol, hyaluronic acid, hybrid safflower (*Carthamus tinctorius*) oil, hydrogenated castor oil, hydrogenated coco-glycerides, hydrogenated coconut oil, hydrogenated lanolin, hydrogenated lecithin, hydrogenated palm glyceride, hydrogenated palm kernel oil, hydrogenated soybean oil, hydrogenated tallow glyceride, hydrogenated vegetable oil, hydrolyzed collagen, hydrolyzed elastin, hydrolyzed glycosaminoglycans, hydrolyzed keratin, hydrolyzed soy protein, hydroxylated lanolin, hydroxyproline, isocetyl stearate, isocetyl stearoyl stearate, isodecyl oleate, isopropyl isostearate, isopropyl lanolate, isopropyl myristate, isopropyl palmitate, isopropyl stearate, isostearamide DEA, isostearic acid, isostearyl lactate, isostearyl neopentanoate, jasmine (*Jasminum officinale*) oil, jojoba (*Buxus chinensis*) oil, kelp, kukui (*Aleurites moluccana*) nut oil, lactamide MEA, laneth-16, laneth-10 acetate, lanolin, lanolin acid, lanolin alcohol, lanolin oil, lanolin wax, lavender (*Lavandula angustifolia*) oil, lecithin, lemon (*Citrus medica limonum*) oil, linoleic acid, linolenic acid, *Macadamia ternifolia* nut oil, maltitol, matricaria (*Chamomilla recutita*) oil, methyl glucose sesquistearate, methylsilanol PCA, mineral oil, mink oil, mortierella oil, myristyl lactate, myristyl myristate, myristyl propionate, neopentyl glycol dicaprylate/dicaprate, octyldodecanol, octyldodecyl myristate, octyldodecyl stearoyl stearate, octyl hydroxystearate, octyl palmitate, octyl salicylate, octyl stearate, oleic acid, olive (*Olea europaea*) oil, orange (*Citrus aurantium dulcis*) oil, palm (*Elaeis guineensis*) oil, palmitic acid, pantethine, panthenol, panthenyl ethyl ether, paraffin, PCA, peach (*Prunus persica*) kernel oil, peanut (*Arachis hypogaea*) oil, PEG-8 C12-18 ester, PEG-15 cocamine, PEG-150 distearate, PEG-60 glyceryl isostearate, PEG-5 glyceryl stearate, PEG-30 glyceryl stearate, PEG-7 hydrogenated castor oil, PEG-40 hydrogenated castor oil, PEG-60 hydrogenated castor oil, PEG-20 methyl glucose sesquistearate, PEG-40 sorbitan peroleate, PEG-5 soy sterol, PEG-10 soy sterol, PEG-2 stearate, PEG-8 stearate, PEG-20 stearate, PEG-32 stearate, PEG-40 stearate, PEG-50 stearate, PEG-100 stearate, PEG-150 stearate, pentadecalactone, peppermint (*Mentha piperita*) oil, petrolatum, phospholipids, plankton extract, polyamino sugar condensate, polyglyceryl-3 diisostearate, polyquaternium-24, polysorbate 20, polysorbate 40, polysorbate 60, polysorbate 80, polysorbate 85, potassium myristate, potassium palmitate, propylene glycol, propylene glycol dicaprylate/dicaprate, propylene glycol dioctanoate, propylene glycol dipelargonate, propylene glycol laurate, propylene glycol stearate, propylene glycol stearate SE, PVP, pyridoxine dipalmitate, retinol, retinyl palmitate, rice (*Oryza sativa*) bran oil, RNA, rosemary (*Rosmarinus officinalis*) oil, rose oil, safflower (*Carthamus tinctorius*) oil, sage (*Salvia officinalis*) oil, sandalwood (*Santalum album*) oil, serine, serum protein, sesame (*Sesamum indicum*) oil, shea butter (*Butyrospermum parkii*), silk powder, sodium chondroitin sulfate, sodium hyaluronate, sodium lactate, sodium palmitate, sodium PCA, sodium polyglutamate, soluble collagen, sorbitan laurate, sorbitan oleate, sorbitan palmitate, sorbitan sesquioleate, sorbitan stearate, sorbitol, soybean (*Glycine soja*) oil, sphingolipids, squalane, squalene, stearamide MEA-stearate, stearic acid, stearoxy dimethicone, stearoxytrimethylsilane, stearyl alcohol, stearyl glycyrrhetinate, stearyl heptanoate, stearyl stearate, sunflower (*Helianthus annuus*) seed oil, sweet almond (*Prunus amygdalus dulcis*) oil, synthetic beeswax, tocopherol, tocopheryl acetate, tocopheryl linoleate, tribehenin, tridecyl neopentanoate, tridecyl stearate, triethanolamine, tristearin, urea, vegetable oil, water, waxes, wheat (*Triticum vulgare*) germ oil, and ylang (*Cananga odorata*) oil.

**c. Antioxidants**

Non-limiting examples of antioxidants that can be used with the compositions of the present invention include acetyl cysteine, ascorbic acid polypeptide, ascorbyl dipalmitate, ascorbyl methylsilanol pectinate, ascorbyl palmitate, ascorbyl stearate, BHA, BHT, t-butyl hydroquinone, cysteine, cysteine HCl, diamylhydroquinone, di-t-butylhydroquinone, dicetyl thiodipropionate, dioleyl tocopheryl methylsilanol, disodium ascorbyl sulfate, distearyl thiodipropionate, ditridecyl thiodipropionate, dodecyl gallate, erythorbic acid, esters of ascorbic acid, ethyl ferulate, ferulic acid, gallic acid esters, hydroquinone, isooctyl thioglycolate, kojic acid, magnesium ascorbate, magnesium ascorbyl phosphate, methylsilanol ascorbate, natural botanical anti-oxidants such as green tea or grape seed extracts, nordihydroguaiaretic acid, octyl gallate, phenylthioglycolic acid, potassium ascorbyl tocopheryl phosphate, potassium sulfite, propyl gallate, quinones, rosmarinic acid, sodium ascorbate, sodium bisulfite, sodium erythorbate, sodium metabisulfite, sodium sulfite, superoxide dismutase, sodium thioglycolate, sorbityl furfural, thiodiglycol, thiodiglycolamide, thiodiglycolic acid, thioglycolic acid, thiolactic acid, thiosalicylic acid, tocophereth-5, tocophereth-10, tocophereth-12, tocophereth-18, tocophereth-50, tocopherol, tocophersolan, tocopheryl acetate, tocopheryl linoleate, tocopheryl nicotinate, tocopheryl succinate, and tris(nonylphenyl)phosphite.

### d. Structuring Agents

In other non-limiting aspects, the compositions of the present invention can include a structuring agent. Structuring agent, in certain aspects, assist in providing rheological characteristics to the composition to contribute to the composition's stability. In other aspects, structuring agents can also function as an emulsifier or surfactant. Non-limiting examples of structuring agents include stearic acid, palmitic acid, stearyl alcohol, cetyl alcohol, behenyl alcohol, stearic acid, palmitic acid, the polyethylene glycol ether of stearyl alcohol having an average of about 1 to about 21 ethylene oxide units, the polyethylene glycol ether of cetyl alcohol having an average of about 1 to about 5 ethylene oxide units, and mixtures thereof.

### e. Emulsifiers

In certain aspects of the present invention, the compositions do not include an emulsifier. In other aspects, however, the compositions can include one or more emulsifiers. Emulsifiers can reduce the interfacial tension between phases and improve the formulation and stability of an emulsion. The emulsifiers can be nonionic, cationic, anionic, and zwitterionic emulsifiers (See McCutcheon's (1986); U.S. Pat. Nos. 5,011,681; 4,421,769; 3,755,560). Non-limiting examples include esters of glycerin, esters of propylene glycol, fatty acid esters of polyethylene glycol, fatty acid esters of polypropylene glycol, esters of sorbitol, esters of sorbitan anhydrides, carboxylic acid copolymers, esters and ethers of glucose, ethoxylated ethers, ethoxylated alcohols, alkyl phosphates, polyoxyethylene fatty ether phosphates, fatty acid amides, acyl lactylates, soaps, TEA stearate, DEA oleth-3 phosphate, polyethylene glycol 20 sorbitan monolaurate (polysorbate 20), polyethylene glycol 5 soya sterol, steareth-2, steareth-20, steareth-21, ceteareth-20, cetearyl glucoside, cetearyl alcohol, C12-13 pareth-3, PPG-2 methyl glucose ether distearate, PPG-5-ceteth-20, bis-PEG/PPG-20/20 dimethicone, ceteth-10, polysorbate 80, cetyl phosphate, potassium cetyl phosphate, diethanolamine cetyl phosphate, polysorbate 60, glyceryl stearate, PEG-100 stearate, arachidyl alcohol, arachidyl glucoside, and mixtures thereof.

### f. Silicone Containing Compounds

In non-limiting aspects, silicone containing compounds include any member of a family of polymeric products whose molecular backbone is made up of alternating silicon and oxygen atoms with side groups attached to the silicon atoms. By varying the -Si-O- chain lengths, side groups, and crosslinking, silicones can be synthesized into a wide variety of materials. They can vary in consistency from liquid to gel to solids.

The silicone containing compounds that can be used in the context of the present invention include those described in this specification or those known to a person of ordinary skill in the art. Non-limiting examples include silicone oils (e.g., volatile and non-volatile oils), gels, and solids. In certain aspects, the silicon containing compounds include silicone oils such as a polyorganosiloxane. Non-limiting examples of polyorganosiloxanes include dimethicone, cyclomethicone, polysilicone-11, phenyl trimethicone, trimethylsilylamodimethicone, stearoxytrimethylsilane, or mixtures of these and other organosiloxane materials in any given ratio in order to achieve the desired consistency and application characteristics depending upon the intended application (e.g., to a particular area such as the skin, hair, or eyes). A "volatile silicone oil" includes a silicone oil have a low heat of vaporization, i.e. normally less than about 50 cal per gram of silicone oil. Non-limiting examples of volatile silicone oils include: cyclomethicones such as Dow Corning 344 Fluid, Dow Corning 345 Fluid, Dow Corning 244 Fluid, and Dow Corning 245 Fluid, Volatile Silicon 7207 (Union Carbide Corp., Danbury, Conn.); low viscosity dimethicones, i.e. dimethicones having a viscosity of about 50 cst or less (e.g., dimethicones such as Dow Corning 200-0.5 cst Fluid). The Dow Corning Fluids are available from Dow Corning Corporation, Midland, Michigan. Cyclomethicone and dimethicone are described in the Third Edition of the CTFA Cosmetic Ingredient Dictionary as cyclic dimethyl polysiloxane compounds and a mixture of fully methylated linear siloxane polymers endblocked with trimethylsiloxy units, respectively. Other non-limiting volatile silicone oils that can be used in the context of the present invention include those available from General Electric Co., Silicone Products Div., Waterford, N.Y. and SWS Silicones Div. of Stauffer Chemical Co., Adrian, Michigan.

## g. Exfoliating Agent

Exfoliating agents include ingredients that remove dead skin cells on the skin's outer surface. These agents may act through mechanical, chemical, and/or other means. Non-limiting examples of mechanical exfoliating agents include abrasives such as pumice, silica, cloth, paper, shells, beads, solid crystals, solid polymers, etc. Non-limiting examples of chemical exfoliating agents include acids and enzyme exfoliants. Acids that can be used as exfoliating agents include, but are not limited to, glycolic acid, lactic acid, citric acid, alpha hydroxy acids, beta hydroxy acids, etc. Other exfoliating agents known to those of skill in the art are also contemplated as being useful within the context of the present invention.

## h. Essential Oils

Essential oils include oils derived from herbs, flowers, trees, and other plants. Such oils are typically present as tiny droplets between the plant's cells, and can be extracted by several method known to those of skill in the art (e.g., steam distilled, enfleurage (i.e., extraction by using fat), maceration, solvent extraction, or mechanical pressing). When these types of oils are exposed to air they tend to evaporate (i.e., a volatile oil). As a result, many essential oils are colorless, but with age they can oxidize and become darker. Essential oils are insoluble in water and are soluble in alcohol, ether, fixed oils (vegetal), and other organic solvents. Typical physical characteristics found in essential oils include boiling points that vary from about 160° to 240° C and densities ranging from about 0.759 to about 1.096.

Essential oils typically are named by the plant from which the oil is found. For example, rose oil or peppermint oil are derived from rose or peppermint plants, respectively. Non-limiting examples of essential oils that can be used in the context of the present invention include sesame oil, macadamia nut oil, tea tree oil, evening primrose oil, Spanish sage oil, Spanish rosemary oil, coriander oil, thyme oil, pimento berries oil, rose oil, anise oil, balsam oil, bergamot oil, rosewood oil, cedar oil, chamomile oil, sage oil, clary sage oil, clove oil, cypress oil, eucalyptus oil, fennel oil, sea fennel oil, frankincense oil, geranium oil, ginger oil, grapefruit oil, jasmine oil, juniper oil, lavender oil, lemon oil, lemongrass oil, lime oil, mandarin oil, marjoram oil, myrrh oil, neroli oil, orange oil, patchouli oil, pepper oil, black pepper oil, petitgrain oil, pine oil, rose otto oil, rosemary oil, sandalwood oil, spearmint oil, spikenard oil, vetiver oil, wintergreen oil, or ylang-ylang. Other essential oils known to those of skill in the art are also contemplated as being useful within the context of the present invention.

## i. Thickening Agents

Thickening agents, including thickener or gelling agents, include substances which that can increase the viscosity of a composition. Thickeners include those that can increase the viscosity of a composition without substantially modifying the efficacy of the active ingredient within the composition. Thickeners can also increase the stability of the compositions of the present invention. In certain aspects of the present invention, thickeners include hydrogenated polyisobutene, trihydroxystearin, ammonium acryloyldimethyltaurate/vp copolymer, or a mixture of them.

Non-limiting examples of additional thickening agents that can be used in the context of the present invention include carboxylic acid polymers, crosslinked polyacrylate polymers, polyacrylamide polymers, polysaccharides, and gums. Examples of carboxylic acid polymers include crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol (see U.S. Pat. Nos. 5,087,445; 4,509,949; 2,798,053; CTFA International Cosmetic Ingredient Dictionary, Fourth edition, 1991, pp. 12 and 80). Examples of commercially available carboxylic acid polymers include carbomers, which are homopolymers of acrylic acid crosslinked with allyl ethers of sucrose or pentaerythritol (e.g., CARBOPOL™ 900 series from B. F. Goodrich).

Non-limiting examples of crosslinked polyacrylate polymers include cationic and nonionic polymers. Examples are described in U.S. Pat. Nos. 5,100,660 ; 4,849,484; 4,835,206; 4,628,078; 4,599,379).

Non-limiting examples of polyacrylamide polymers (including nonionic polyacrylamide polymers including substituted branched or unbranched polymers) include polyacrylamide, isoparaffin and laureth-7, multi-block copolymers of acrylamides and substituted acrylamides with acrylic acids and substituted acrylic acids.

Non-limiting examples of polysaccharides include cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Another example is an alkyl substituted cellulose where the hydroxy groups of the cellulose polymer is hydroxyalkylated (preferably hydroxy ethylated or hydroxypropylated) to form a hydroxyalkylated cellulose which is then further modified with a C10 -C30 straight chain or branched chain alkyl group through an ether linkage. Typically these polymers are ethers of C10-C30 straight or branched chain alcohols with hydroxyalkylcelluloses. Other useful polysaccharides include scleroglucans comprising a linear chain of (1-3) linked glucose units with a (1-6) linked glucose every three units.

Non-limiting examples of gums that can be used with the present invention include acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluronic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboxymethyl dextran, sodium carrageenan, tragacanth gum, xanthan gum, and mixtures thereof.

**j. Preservatives**

Non-limiting examples of preservatives that can be used in the context of the present invention include quaternary ammonium preservatives such as polyquaternium-1 and benzalkonium halides (e.g., benzalkonium chloride ("BAC") and benzalkonium bromide), parabens (e.g., methylparabens and propylparabens), phenoxyethanol, benzyl alcohol, chlorobutanol, phenol, sorbic acid, thimerosal or combinations thereof.

**2. Pharmaceutical Ingredients**

Pharmaceutical active agents are also contemplated as being useful with the compositions of the present invention. Non-limiting examples of pharmaceutical active agents include anti-acne agents, agents used to treat rosacea, analgesics, anesthetics, anorectals, antihistamines, anti-inflammatory agents including non-steroidal anti-inflammatory drugs, antibiotics, antifungals, antivirals, antimicrobials, anti-cancer actives, scabicides, pediculicides, antineoplastics, antiperspirants, antipruritics, antpsoriatic agents, anti seborrheic agents, biologically active proteins and peptides, burn treatment agents, cauterizing agents, depigmenting agents, depilatories, diaper rash treatment agents, enzymes, hair growth stimulants, hair growth retardants including DFMO and its salts and analogs, hemostatics, kerotolytics, canker sore treatment agents, cold sore treatment agents, dental and periodontal treatment agents, photosensitizing actives, skin protectant/barrier agents, steroids including hormones and corticosteroids, sunburn treatment agents, sunscreens, transdermal actives, nasal actives, vaginal actives, wart treatment agents, wound treatment agents, wound healing agents, etc.

**F. Kits**

Kits are also contemplated as being used in certain aspects of the present invention. For instance, compositions of the present invention can be included in a kit. A kit can include a container. Containers can include a bottle, a metal tube, a laminate tube, a plastic tube, a dispenser, a pressurized container, a barrier container, a package, a compartment, a lipstick container, a compact container, cosmetic pans that can hold cosmetic compositions, or other types of containers such as injection or blow-molded plastic containers into which the dispersions or compositions or desired bottles, dispensers, or packages are retained. The kit and/or container can include indicia on its surface. The indicia, for example, can be a word, a phrase, an abbreviation, a picture, or a symbol.

The containers can dispense a pre-determined amount of the composition. In other embodiments, the container can be squeezed (e.g., metal, laminate, or plastic tube) to dispense a desired amount of the composition. The composition can be dispensed as a spray, an aerosol, a liquid, a fluid, or a semi-solid. The containers can have spray, pump, or squeeze mechanisms. A kit can also include instructions for employing the kit components as well the use of any other compositions included in the container. Instructions can include an explanation of how to apply, use, and maintain the compositions.

**EXAMPLES**

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

**EXAMPLE 1**

Combinations of active ingredients disclosed herein can be included in a wide-range of topical product formulations for skin and/or hair. Combinations from Example 1 may be prepared as topical skin or hair compositions. In some aspects, the combination in Table 1 may be prepared as a facial emulsion for day time use. In some aspects, the combination in Table 2 may be prepared as a serum. In some aspects, the combination in Table 3 may be prepared as an eye gel. In some aspects, the combination of Table 4 may be prepared as a freshener. In some aspects, the combination of Table 5 may be prepared as a cleanser. In some aspects, the combination of Table 6 may be prepared as a moisturizer.

All of the compositions disclosed and claimed herein can be made and executed without undue experimentation in light of the present disclosure. More specifically, it will be apparent that certain agents which are both chemically and physiologically related may be substituted for the agents described herein while the same or similar results would be achieved.

**TABLE 1**

| Facial cream (not according to the invention) |
| --- |
| **Ingredient** |
| *Myrothamnus flabellifolia* leaf/stem extract |
| Saccharide isomerate |

**TABLE 2**

| Serum (not according to the invention) |
| --- |
| **Ingredient** |
| *Myrothamnus flabellifolia* leaf/stem extract |
| Saccharide isomerate |
| *Opuntia tuna* fruit extract (optional) |

**TABLE 3**

| Eye Gel (not according to the invention) |
| --- |
| **Ingredient** |
| *Myrothamnus flabellifolia* leaf/stem extract |
| Saccharide isomerate |
| *Opuntia tuna* fruit extract (optional) |

**TABLE 4**

| Freshener |
| --- |
| **Ingredient** |
| *Myrothamnus flabellifolia* leaf/stem extract |
| *Alteromonas* ferment extract |

**TABLE 5**

| Cleanser |
| --- |
| **Ingredient** |
| *Myrothamnus flabellifolia* leaf/stem extract |
| *Alteromonas* ferment extract |
| *Opuntia tuna* fruit extract (optional) |

**TABLE 6**

| Moisturizer (not according to the invention) |
|---|
| **Ingredient** |
| *Myrothamnus flabellifolia* leaf/stem extract |
| Saccharide isomerate |

**TABLE 7**

| Mask (not according to the invention) |
|---|
| **Ingredient** |
| *Myrothamnus flabellifolia* leaf/stem extract |
| Saccharide isomerate |

**TABLE 8**

| Mask |
|---|
| **Ingredient** |
| *Myrothamnus flabellifolia* leaf/stem extract |
| *Alteromonas* ferment extract |

**EXAMPLE 2**

Tables 9 and 10 describe generic formulations or skin testing formulations in which an active ingredient can be incorporated into. These formulations can also be used to determine the types of skin benefits that can be attributed to the active ingredient. These formulations are prepared in such a manner that any resulting skin benefit from topical application of the formula to skin can be directly attributed to the active ingredient being tested. In the context of aspects of the present invention, the active ingredient that can be tested can be *Myrothamnus flabellifolia* leaf/stem extract, plankton extract, *Alteromonas* ferment extract, malachite extract, and/or *Opuntia tuna* fruit extract, or any combination thereof, or all of such active ingredients, or at least 1, 2, 3, 4, and/or 5 of such active ingredients. It should be recognized that other standard testing vehicles can also be used to determine the skin benefit properties of active ingredient and that the following formulations are non-limiting testing vehicles.

**TABLE 9***

| Ingredient | % Concentration (by weight) |
|---|---|
| **Phase A** | |
| Water | 84.80 |
| Xanthan gum | 0.1 |
| M-paraben | 0.15 |
| P-paraben | 0.1 |
| Citric acid | 0.1 |
| **Phase B** | |
| Cetyl alcohol | 4.0 |
| Glyceryl stearate + PEG 100 | 4.0 |
| Octyl palmitate | 4.0 |

(continued)

| Phase B | |
|---|---|
| Dimethicone | 1.0 |
| Tocopheryl acetate | 0.2 |
| Phase C | |
| Active Ingredient** | 2.0 |
| TOTAL | 100 |

\* Procedure for making composition: Sprinkle Xanthan gum in water and mix for 10 min. Subsequently, add all ingredients in phase A and heat to 70-75°C. Add all items in phase B to separate beaker and heat to 70-75°C. Mix phases A and B at 70-75°C. Continue mixing and allow composition to cool to 30°C. Subsequently, add phase C ingredient while mixing.

\*\* The active ingredients identified throughout this specification can be incorporated into a composition as the active ingredient. The active ingredients can be individually used or combined in this composition. The concentration ranges of the active ingredients (or combination of active ingredients) can be modified as desired or needed by increasing or decreasing the amount of water.

**TABLE 10\***

| Ingredient | % Concentration (by weight) |
|---|---|
| Phase A | |
| Water | 78.6 |
| M-paraben | 0.2 |
| P-paraben | 0.1 |
| Na$_2$ EDTA | 0.1 |
| Shea butter | 4.5 |
| Petrolatum | 4.5 |
| Glycerin | 4.0 |
| Propylene Glycol | 2.0 |
| FINSOLV® TN | 2.0 |
| Phase B | |
| SEPIGEL™ 305 | 2.0 |
| Phase C | |
| Active Ingredient ** | 2.0 |
| TOTAL | 100 |

\* Add ingredients in phase A to beaker and heat to 70-75°C while mixing. Subsequently, add the phase B ingredient with phase A and cool to 30°C with mixing. Subsequently, add phase C ingredient while mixing.

\*\* The active ingredients identified throughout this specification can be incorporated into a composition as the active ingredient. The active ingredients can be individually used or combined in this composition. The concentration ranges of the active ingredients (or combination of active ingredients) can be modified as desired or needed by increasing or decreasing the amount of water.

**EXAMPLE 3**

The formulations represented in Table 11-16 are non-limiting examples of the types of formulations that can be prepared in the context of the present invention. Any standard method can be used to prepare such formulations.

For instance, simple mixing of the ingredients in a beaker can be used. One should mix such ingredients and add heat as necessary to obtain a homogenous composition. The active ingredients that can be used in the formulations include *Myrothamnus flabellifolia* leaf/stem extract and *Alteromonas* ferment extract, and, optionally saccharide isomerate, , malachite extract, and/or *Opuntia tuna* fruit extract.

Table 11 includes a non-limiting example of a composition of the present invention. The composition can be formulated into an emulsion (e.g., o/w, w/o, o/w/o, w/o/w, etc.) and the additional ingredients identified throughout the specification can be included into the Table 11 composition (e.g., by adjusting the water content of the composition). Further, the concentration ranges of the ingredients identified in Table 11 can vary depending on a desired formulation (e.g., cream, lotion, moisturizer cleanser, etc.).

**TABLE 11**

| Ingredient | % Concentration (by weight) |
|---|---|
| Water | q.s. |
| Active Ingredient* | 0.1% to 10% |
| Glycerin | 3 to 40% |
| Butylene glycol | 0.0001 to 10% |
| Propylene glycol | 0.0001 to 10% |
| Phenoxyethanol | 0.0001 to 10% |
| Disodium EDTA | 0.0001 to 10% |
| Steareth-20 | 0.0001 to 10% |
| Chlorhexidine Digluconate | 0.0001 to 10% |
| Potassium Sorbate | 0.0001 to 10% |
| Preservative* * | 0.0001 to 2% |
| **TOTAL** | **100** |

\* The active ingredients identified throughout this specification can be incorporated into a composition as the active ingredient. The active ingredients can be individually used or combined in this composition. The concentration ranges of the active ingredients (or combination of active ingredients) can be modified as desired or needed by increasing or decreasing the amount of water.

\*\*Any preservative can be used identified in the specification or those known in the art.

Table 12 includes a non-limiting example of a composition of the present invention. The composition can be formulated into an emulsion (e.g., o/w, w/o, o/w/o, w/o/w, etc.) and the additional ingredients identified throughout the specification can be included into the Table 12 composition (e.g., by adjusting the water content of the composition). Further, the concentration ranges of the ingredients identified in Table 12 can vary depending on a desired formulation (e.g., cream, lotion, moisturizer cleanser, etc.).

**TABLE 12**

| Ingredient | % Concentration (by weight) |
|---|---|
| Water | q.s. |
| Active Ingredient* | 0.1% to 10% |
| Dimethicone | 0.0001 to 10% |
| Triethanolamine | 0.0001 to 10% |
| Phenonip | 0.0001 to 10% |
| Betaine | 0.0001 to 10% |
| Disodium EDTA | 0.0001 to 10% |
| Tocopheryl acetate | 0.0001 to 10% |

(continued)

| Ingredient | % Concentration (by weight) |
|---|---|
| PRODEW® 400 | 0.0001 to 10% |
| Preservative* * | 0.0001 to 2% |
| **TOTAL** | **100** |

* The active ingredients identified throughout this specification can be incorporated into a composition as the active ingredient. The active ingredients can be individually used or combined in this composition. The concentration ranges of the active ingredients (or combination of active ingredients) can be modified as desired or needed by increasing or decreasing the amount of water.
**Any preservative can be used identified in the specification or those known in the art.

Table 13 includes a non-limiting example of a composition of the present invention. The composition can be formulated into an emulsion (e.g., o/w, w/o, o/w/o, w/o/w, etc.) and the additional ingredients identified throughout the specification can be included into the Table 13 composition (e.g., by adjusting the water content of the composition). Further, the concentration ranges of the ingredients identified in Table 13 can vary depending on a desired formulation (e.g., cream, lotion, moisturizer cleanser, etc.). In particular embodiments, the Table 13 composition can be a moisturizer.

**Table 13**

| Ingredient | % Concentration (by weight) |
|---|---|
| Water | q.s. |
| Active Ingredient * | 0.1% to 10% |
| Glycerin | 0.0001 to 10% |
| Pentylene Glycol | 0.0001 to 10% |
| Capryl Glycol | 0.0001 to 10% |
| Disodium EDTA | 0.0001 to 10% |
| Capric/Caprylic Triglyceride | 0.0001 to 10% |
| Lipex 205 (Shea Butter) | 0.0001 to 10% |
| Squalane | 0.0001 to 10% |
| Cetyl Alcohol | 0.0001 to 10% |
| Dimethicone | 0.0001 to 10% |
| Ceramide II | 0.0001 to 10% |
| Stearic Acid | 0.0001 to 10% |
| Super Sterol Ester | 0.0001 to 10% |
| Arlacel 165 | 0.0001 to 10% |
| Simulgel 600 | 0.0001 to 10% |
| **TOTAL** | **100** |

* The active ingredients identified throughout this specification can be incorporated into composition as the active ingredient. The active ingredients can be individually used or combined in this composition. The concentration ranges of the active ingredients (or combination of active ingredients) can be modified as desired or needed by increasing or decreasing the amount of water.

Table 14 includes a non-limiting example of a composition of the present invention. The composition can be formulated into an emulsion (e.g., o/w, w/o, o/w/o, w/o/w, etc.) and the additional ingredients identified throughout the specification can be included into the Table 14 composition (e.g., by adjusting the water content of the composition). Further, the concentration ranges of the ingredients identified in Table 14 can vary depending on a desired formulation (e.g., cream, lotion, moisturizer cleanser, etc.). In particular embodiments, the Table 14 composition can be a moisturizer.

**Table 14**

| Ingredient | % Concentration (by weight) |
|---|---|
| Water | q.s. |
| Active Ingredient * | 0.1% to 10% |
| Glycerin | 0.0001 to 10% |
| Pentylene Glycol | 0.0001 to 10% |
| Capryl Glycol | 0.0001 to 10% |
| Disodium EDTA | 0.0001 to 10% |
| Petrolatum | 0.0001 to 10% |
| Squalane | 0.0001 to 10% |
| Cetyl Alcohol | 0.0001 to 10% |
| ARLACEL™ 165 | 0.0001 to 10% |
| Dimethicone | 0.0001 to 10% |
| SIMULGEL™ 600 | 0.0001 to 10% |
| **TOTAL** | **100** |
| * The active ingredients identified throughout this specification can be incorporated into a composition as the active ingredient. The active ingredients can be individually used or combined in this composition. The concentration ranges of the active ingredients (or combination of active ingredients) can be modified as desired or needed by increasing or decreasing the amount of water. | |

Table 15 includes a non-limiting example of a composition of the present invention. The composition can be formulated into an emulsion (e.g., o/w, w/o, o/w/o, w/o/w, etc.) and the additional ingredients identified throughout the specification can be included into the Table 15 composition (e.g., by adjusting the water content of composition). Further, the concentration ranges of the ingredients identified in Table 15 can vary depending on a desired formulation (e.g., cream, lotion, moisturizer cleanser, etc.). In particular embodiments, the Table 15 composition can be a sunscreen lotion.

**Table 15**

| Ingredient | % Concentration (by weight) |
|---|---|
| Water | q.s. |
| Active Ingredient* | 0.1% to 10% |
| Xanthan Gum | 0.0001 to 10% |
| Disodium EDTA | 0.0001 to 10% |
| Pentylene Glycol | 0.0001 to 10% |
| Capryl Glycol | 0.0001 to 10% |
| PEMULEN™ TR-1 | 0.0001 to 10% |
| Triethanolamine | 0.0001 to 10% |
| PVP/Hexadecene Copolymer | 0.0001 to 10% |
| FINSOLV® TN | 10 to 30% |
| Sorbitan Isostearate | 0.0001 to 10% |
| Sunscreen Ingredient** | 2 to 25% |

(continued)

| Ingredient | % Concentration (by weight) |
|---|---|
| TOTAL | 100 |

* The active ingredients identified throughout this specification can be incorporated into a composition as the active ingredient. The active ingredients can be individually used or combined in this composition. The concentration ranges of the active ingredients (or combination of active ingredients) can be modified as desired or needed by increasing or decreasing the amount of water.

**Sunscreen ingredient can be any sunscreen ingredient, or combination of such ingredients, identified in the specification (e.g. UV absorbing and/or reflecting agents) or known to those of ordinary skill in the art. In one embodiment, the sunscreen ingredient is a combination of zinc oxide and titanium dioxide.

Table 16 includes a non-limiting example of a composition of the present invention. The additional ingredients identified throughout the specification can be included into the Table 16 composition (e.g., by adjusting the water content of composition). Further, the concentration ranges of the ingredients identified in Table 16 can vary depending on a desired formulation (e.g., cream, lotion, moisturizer cleanser, etc.). In particular embodiments, the Table 16 composition can be a cleanser.

Table 16

| Ingredient | % Concentration (by weight) |
|---|---|
| Water | q.s. |
| Active Ingredient* | 0.1% to 10% |
| Disodium EDTA | 0.0001 to 10% |
| Citric Acid | 0.0001 to 10% |
| Pentylene Glycol | 0.0001 to 10% |
| Capryl Glycol | 0.0001 to 10% |
| Sodium methyl cocoyl taurate | 10 to 30% |
| Sodium cocoamphodiacetate | 1 to 10% |
| TOTAL | 100 |

* The active ingredients identified throughout this specification can be incorporated into a composition as the active ingredient. The active ingredients can be individually used or combined in this composition. The concentration ranges of the active ingredients (or combination of active ingredients) can be modified as desired or needed by increasing or decreasing the amount of water.

## EXAMPLE 4

Formulations having combinations of active ingredients disclosed herein from Example 1 were prepared as topical skin and/or hair compositions. The formulations in Tables 17, 18 and 19 were prepared as SPF 25 (Sun Protection Factor 25) facial emulsions and are not according to the invention. The formulation in Table 20 was prepared as a facial serum. The formulations in Tables 21 and 22 were prepared as eye gels and are not according to the invention. The formulation in Table 23 was prepared as a freshener according to the invention. Table 24 was prepared as a cleanser according to the invention. Table 25 was prepared as a moisturizer and is not according to the invention. Tables 26 (not according to the invention) and 27 (according to the invention) were prepared as masks. Table 28 also disclosed herein was prepared as a serum.

TABLE 17* (not according to the invention)

| Ingredient | % Concentration (by weight) |
|---|---|
| Water | 55.6 |
| Octinoxate | 6.8 |

(continued)

| Ingredient | % Concentration (by weight) |
|---|---|
| Zinc Oxide | 4.5 |
| C12-15 Alkyl Benzoate | 4.4 |
| Ethylhexyl Salicylate | 4.5 |
| Isododecane | 4.0 |
| Propanediol | 2.1 |
| Ensulizole | 2.0 |
| Glycerin | 2.0 |
| Nylon-12 | 2.0 |
| Propylene Glycol | 1.5 |
| Arginine | 1.2 |
| Betaine | 1.0 |
| Glyceryl stearate | 0.9 |
| Dimethicone | 0.8 |
| Cetearyl alcohol | 0.8 |
| Hydroxyacetophenone | 0.8 |
| PEG-100 Stearate | 0.6 |
| Titanium dioxide | 0.6 |
| 1,2-hexanediol | 0.5 |
| MICA | 0.4 |
| Hydroxyethyl acrylate/sodium acryloyldimethyl Taurate | 0.4 |
| Hydroxyethylcellulose | 0.3 |
| Polyhydroxystearic acid | 0.3 |
| Squalane | 0.3 |
| Ceteareth-20 | 0.2 |
| Disodium EDTA | 0.2 |
| Glyceryl polyacrylate | 0.2 |
| Triethoxycaprylylsilane | 0.1 |
| Bisabolol | 0.1 |
| Dipotassium Glycyrrhizate | 0.1 |
| Tocopheryl acetate | 0.1 |
| *Myrothamnus flabellifolia* leaf/stem extract | 0.03 |
| Saccharide isomerate | 0.01 |
| Excipients** | q.s. |

*The formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition.

**Excipients can be added, for example, to modify the rheological properties of the composition. Alternatively, the amount of water can be varied so long as the amount of water in the composition is at least 35% w/w, and preferably between 40 to 80% w/w.

**TABLE 18\* (not according to the invention)**

| Ingredient | % Concentration (by weight) |
| --- | --- |
| Water | 60.1 |
| Homosalate | 7.0 |
| Ethylhexyl Salicylate | 4.5 |
| Avobenzone | 3.0 |
| Glycerin | 3.0 |
| Styrene/Acrylates copolymer | 2.4 |
| C12-15 Alkyl Benzoate | 2.1 |
| Butylene glycol | 2.0 |
| Dicaprylyl carbonate | 2.0 |
| Octocrylene | 2.0 |
| Ceteareth-25 | 1.3 |
| Magnesium aluminum silicate | 1.0 |
| Dipropylene glycol dibenzoate | 0.8 |
| Hydroxyethyl acrylate/sodium acryloyldimethyl Taurate | 0.8 |
| Phenoxyethanol | 0.6 |
| Propanediol | 0.6 |
| Disodium ethylene dicocamide PEG-15 disulfate | 0.5 |
| Pentylene glycol | 0.5 |
| Tocopheryl acetate | 0.5 |
| Caprylyl glycol | 0.4 |
| Squalane | 0.5 |
| Hydrogenated lecithin | 0.2 |
| Xanthan Gum | 0.2 |
| PPG-15 Stearyl ether benzoate | 0.2 |
| Disodium EDTA | 0.1 |
| *Myrothamnus flabellifolia* leaf/stem extract | 0.03 |
| Saccharide isomerate | 0.01 |
| Excipients\*\* | q.s. |

\*The formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition.

\*\*Excipients can be added, for example, to modify the rheological properties of the composition. Alternatively, the amount of water can be varied so long as the amount of water in the composition is at least 35% w/w, and preferably between 40 to 80% w/w.

**TABLE 19\* (not according to the invention)**

| Ingredient | % Concentration (by weight) |
| --- | --- |
| Water | 58.0 |

(continued)

| Ingredient | % Concentration (by weight) |
|---|---|
| Homosalate | 9.0 |
| Ethylhexyl Salicylate | 4.5 |
| Oxybenzone | 4.0 |
| Avobenzone | 3.0 |
| Glycerin | 3.0 |
| Styrene/Acrylates copolymer | 2.4 |
| C12-15 Alkyl Benzoate | 2.1 |
| Butylene glycol | 2.0 |
| Dicaprylyl carbonate | 2.0 |
| Octocrylene | 2.0 |
| Polysilicone-11 | 1.9 |
| Ceteareth-25 | 1.3 |
| Dimethicone | 1.1 |
| Magnesium aluminum silicate | 1.0 |
| Hydroxyethyl acrylate/sodium acryloyldimethyl Taurate | 0.8 |
| Dipropylene glycol dibenzoate | 0.8 |
| Phenoxyethanol | 0.6 |
| Propanediol | 0.6 |
| Squalane | 0.5 |
| Disodium ethylene dicocamide PEG-15 disulfate | 0.5 |
| Pentylene glycol | 0.5 |
| Tocopheryl acetate | 0.5 |
| Caprylyl glycol | 0.4 |
| Cetearyl alcohol | 0.3 |
| Bisabolol | 0.2 |
| Hydrogenated lecithin | 0.2 |
| Xanthan Gum | 0.2 |
| PPG-15 Stearyl ether benzoate | 0.2 |
| Disodium EDTA | 0.1 |
| *Myrothamnus flabellifolia* leaf/stem extract | 0.03 |
| Saccharide isomerate | 0.01 |
| Excipients** | q.s. |

*The formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition.
**Excipients can be added, for example, to modify the rheological properties of the composition. Alternatively, the amount of water can be varied so long as the amount of water in the composition is at least 35% w/w, and preferably between 40 to 80% w/w.

**TABLE 20* (not according to the invention)**

| Ingredient | % Concentration (by weight) |
|---|---|
| Water | 72 |
| Butylene Glycol | 6.0 |
| Alcohol Denat | 4.6 |
| Glycerin | 3.5 |
| Polymethylsilsesquioxane | 2.5 |
| Isohexadecane | 2.0 |
| PEG-32 | 1.0 |
| Phenoxyethanol | 0.8 |
| PEG-8 | 0.7 |
| Propanediol | 0.6 |
| PEG-7 Glyceryl cocoate | 0.4 |
| Acacia Senegal gum extract | 0.3 |
| Triethanolamine | 0.3 |
| Acrylates/C 10-30 Alkyl acrylate crosspolymer | 0.3 |
| Disodium EDTA | 0.2 |
| Xanthan Gum | 0.1 |
| *Myrothamnus flabellifolia* leaf/stem extract | 0.03 |
| Saccharide isomerate | 0.01 |
| Excipients** | q.s. |

*The formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition.

**Excipients can be added, for example, to modify the rheological properties of the composition. Alternatively, the amount of water can be varied so long as the amount of water in the composition is at least 35% w/w, and preferably between 55 to 85% w/w.

**TABLE 21* (not according to the invention)**

| Ingredient | % Concentration (by weight) |
|---|---|
| Water | 85 |
| Glycerin | 5.4 |
| Propanediol | 2.6 |
| Butylene Glycol | 2.5 |
| Glycereth-26 | 2.0 |
| 1,2-Hexanediol | 1.0 |
| Triethanolamine | 0.6 |
| Hydroxyacetophenone | 0.5 |
| Acrylates/C 10-30 Alkyl acrylate crosspolymer | 0.3 |
| Hydroxyethylcellulose | 0.2 |

(continued)

| Ingredient | % Concentration (by weight) |
|---|---|
| Sodium polyacrylate | 0.2 |
| Disodium EDTA | 0.1 |
| *Myrothamnus flabellifolia* leaf/stem extract | 0.03 |
| Saccharide isomerate | 0.01 |
| *Opuntia tuna* fruit extract (optional) | 0.0005 |
| Excipients** | q.s. |

*The formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition.
**Excipients can be added, for example, to modify the rheological properties of the composition. Alternatively, the amount of water can be varied so long as the amount of water in the composition is at least 35% w/w, and preferably between 70 to 95% w/w.

**TABLE 22* (not according to the invention)**

| Ingredient | % Concentration (by weight) |
|---|---|
| Water | 86 |
| Glycerin | 5.4 |
| Butylene Glycol | 4.0 |
| Glycereth-26 | 2.0 |
| Propylene glycol | 0.6 |
| Propanediol | 0.6 |
| Triethanolamine | 0.6 |
| 1,2-Hexanediol (optional) | 1.0 |
| Acrylates/C 10-30 Alkyl acrylate crosspolymer | 0.3 |
| Diazolidinyl urea | 0.3 |
| Hydroxyethylcellulose | 0.2 |
| Sodium polyacrylate | 0.2 |
| Methylparaben | 0.1 |
| Disodium EDTA | 0.1 |
| *Myrothamnus flabellifolia* leaf/stem extract | 0.03 |
| Saccharide isomerate | 0.01 |
| *Opuntia tuna* fruit extract (optional) | 0.0005 |
| Excipients** | q.s. |

*The formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition.
**Excipients can be added, for example, to modify the rheological properties of the composition. Alternatively, the amount of water can be varied so long as the amount of water in the composition is at least 35% w/w, and preferably between 70 to 95% w/w.

### TABLE 23*

| Ingredient | % Concentration (by weight) |
|---|---|
| Water | 87 |
| Butylene Glycol | 2.5 or 6.3 |
| Glycerin | 4.5 |
| Caprylyl Glycol | 0.5 |
| PPG-5-Ceteth-20 | 0.5 |
| 1,2-Hexanediol | 0.5 |
| Sodium citrate | 0.4 |
| *Alteromonas* ferment extract | 0.01 |
| *Myrothamnus flabellifolia* leaf/stem extract | 0.003 |
| Excipients** | q.s. |
| *The formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition.<br>**Excipients can be added, for example, to modify the rheological properties of the composition. Alternatively, the amount of water can be varied so long as the amount of water in the composition is at least 35% w/w, and preferably between 70 to 95% w/w. | |

### TABLE 24*

| Ingredient | % Concentration (by weight) |
|---|---|
| Water | 65 |
| Glycerin | 12 |
| Disodium cocoamphodiacetate | 4.0 |
| Tea-lauryl sulfate | 3.6 |
| Potassium myristate | 3.5 |
| Potassium cocoyl glycinate | 2.9 |
| Acrylates copolymer | 2.3 |
| Glyceryl stearate | 1.5 |
| Cocamidopropyl betaine | 1.2 |
| PEG-100 Stearate | 1.0 |
| PEG-120 Methyl glucose dioleate | 0.7 |
| Propylene glycol | 0.6 |
| Butylene glycol | 0.3 |
| Diazolidinyl urea | 0.3 |
| Disodium EDTA | 0.2 |
| Sodium chloride | 0.1 |
| Methylparaben | 0.1 |
| Citric acid | 0.1 |
| *Alteromonas* ferment extract | 0.01 |

(continued)

| Ingredient | % Concentration (by weight) |
|---|---|
| *Myrothamnus flabellifolia* leaf/stem extract | 0.003 |
| *Opuntia tuna* fruit extract (optional) | 0.0005 |
| Excipients** | q.s. |

*The formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition.
**Excipients can be added, for example, to modify the rheological properties of the composition. Alternatively, the amount of water can be varied so long as the amount of water in the composition is at least 35% w/w, and preferably between 40 to 80% w/w.

**TABLE 25* (not according to the invention)**

| Ingredient | % Concentration (by weight) |
|---|---|
| Water | 74 |
| Glycerin | 5.0 |
| Caprylic/Capric triglyceride | 4.0 |
| C12-15 Alkyl benzoate | 3.0 |
| Cetyl alcohol | 2.5 |
| Stearyl alcohol | 2.5 |
| Butylene glycol | 2.0 |
| *Butyrospermum parkii* (Shea) butter | 1.5 |
| Dimethicone | 1.0 |
| Glyceryl stearate | 0.9 |
| Acrylamide/Sodium acryloyldimethyltaurate copolymer | 0.8 |
| PEG-100 Stearate | 0.6 |
| Propanediol | 0.6 |
| Caprylyl glycol | 0.5 |
| Isohexadecane | 0.5 |
| Polysorbate 80 | 0.2 |
| Ceramide NG | 0.1 |
| *Myrothamnus flabellifolia* leaf/stem extract | 0.03 |
| Saccharide isomerate | 0.01 |
| Excipients** | q.s. |

*The formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition.
**Excipients can be added, for example, to modify the rheological properties of the composition. Alternatively, the amount of water can be varied so long as the amount of water in the composition is at least 35% w/w, and preferably between 65 to 85% w/w.

**TABLE 26\* (not according to the invention)**

| Ingredient | % Concentration (by weight) |
|---|---|
| Water | 41 |
| Propanediol | 21 |
| *Butyrospermum parkii* (Shea) butter | 12.5 |
| Glycerin | 9.0 |
| Cetearyl alcohol | 6.0 |
| Glyceryl stearate | 2.0 |
| Lauroyl lysine | 1.8 |
| *Olea europaea* (olive) fruit oil | 1.5 |
| *Prunus armeniaca* (apricot) kernel oil | 1.5 |
| Cetearyl glucoside | 1.0 |
| Sclerotium gum | 0.6 |
| Squalane | 0.5 |
| Phenoxyethanol | 0.4 |
| Dimethicone | 0.3 |
| Tocopheryl acetate | 0.3 |
| Xanthan gum | 0.3 |
| *Oryza sativa* (rice) bran oil | 0.2 |
| Disodium EDTA | 0.1 |
| *Myrothamnus flabellifolia* leaf/stem extract | 0.03 |
| Saccharide isomerate | 0.01 |
| Excipients\*\* | q.s. |

\*The formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition.
\*\*Excipients can be added, for example, to modify the rheological properties of the composition. Alternatively, the amount of water can be varied so long as the amount of water in the composition is at least 35% w/w, and preferably between 35 to 60% w/w.

**TABLE 27\***

| Ingredient | % Concentration (by weight) |
|---|---|
| Water | 48 |
| Kaolin | 30 |
| Glycerin | 6.0 |
| Propylene glycol | 5.0 |
| Magnesium aluminum silicate | 4.0 |
| Bentonite | 3.0 |
| Iron oxides | 1.2 |
| Phenoxyethanol | 0.6 |

(continued)

| Ingredient | % Concentration (by weight) |
|---|---|
| Salicylic acid | 0.5 |
| Triethanolamine | 0.4 |
| Xanthan gum | 0.4 |
| Butylene glycol | 0.3 |
| Carrageenan | 0.2 |
| Disodium EDTA | 0.1 |
| *Alteromonas* ferment extract | 0.01 |
| *Myrothamnus flabellifolia* leaf/stem extract | 0.003 |
| Excipients** | q.s. |

*The formulation can be prepared by mixing the ingredients in a beaker under heat 70-75° C until homogenous. Subsequently, the formulation can be cooled to standing room temperature (20-25°C). Further, and if desired, additional ingredients can be added, for example, to modify the rheological properties of the composition.

**Excipients can be added, for example, to modify the rheological properties of the composition. Alternatively, the amount of water can be varied so long as the amount of water in the composition is at least 35% w/w, and preferably between 35 to 60% w/w.

**TABLE 28* (not according to the invention)**

| Ingredient | % Concentration (by weight) |
|---|---|
| Water | 67 |
| Alcohol | 6.5 |
| Butylene glycol | 6.0 |
| Glycerin | 5.8 |
| Polymethylsilsesquioxane | 4.0 |
| Caprylic/capric/succinic triglyceride | 3.0 |
| Isohexadecane | 2.0 |
| PEG-60 hydrogenated castor oil | 1.5 |
| PEG-32 | 1.0 |
| PEG-8 | 0.7 |
| Phenoxyethanol | 0.6 |
| Propanediol | 0.6 |
| Triethanolamine | 0.3 |
| *Acacia senegal* gum extract | 0.3 |
| Acrylates/C10-30 Alkyl acrylate crosspolymer | 0.3 |
| Disodium EDTA | 0.2 |
| Carbomer | 0.1 |
| Xanthan gum | 0.1 |
| *Myrothamnus flabellifolia* leaf/stem extract | 0.03 |
| Saccharide isomerate | 0.01 |
| *Opuntia tuna* fruit extract (optional) | 0.0005 |

(continued)

| Ingredient | % Concentration (by weight) |
|---|---|
| Excipients** | q.s. |

*The formulation can be prepared by mixing the ingredients in a beaker under heat 70-75°C until homogenous. Subsequently, the formulation can be cooled to standing room temperature (20-25°C). Further, and if desired, additional ingredients can be added, for example, to modify the rheological properties of the composition.
**Excipients can be added, for example, to modify the rheological properties of the composition. Alternatively, the amount of water can be varied so long as the amount of water in the composition is at least 35% w/w, and preferably between 55 to 85% w/w.

## EXAMPLE 5

### (In Vitro Efficacy of Ingredients)

The efficacy of the ingredients were determined by the following methods. The following are non-limiting assays that can be used in the context of the present invention. It should be recognized that other testing procedures can be used, including, for example, objective and subjective procedures.

It was determined that *Myrothamnus flabellifolia* extract inhibits TNF-$\alpha$, reduces nitric oxide synthase, and blocks the accumulation of fine particles (e.g., PM 2.5) on the skin. It was also determined that saccharide isomerate inhibits TNF-$\alpha$, increase production of barrier proteins of Occludin-1 and Filaggrin, and reduces nitric oxide synthase. It was also found that *Alteromonas* ferment extract chelates heavy metals, such as cadmium and lead. A summary of quantitative results is found in Table 29 and the methods used to determine the properties of the ingredients are provided below.

TABLE 29

| Assay | Ingredient | Activity |
|---|---|---|
| Inhibition of TNF-$\alpha$ | *Myrothamnus flabellifolia* extract<br>Saccharide isomerate | -76%<br>-88% |
| | *Myrothamnus flabellifolia* extract | -36% |
| Inhibition of Nitric Oxide | Saccharide isomerate | -46% |
| Increase of Occludin-1 | Saccharide isomerate | +171% |
| Increase of Filaggrin | Saccharide isomerate | +197% |
| Heavy Metal Chelation | *Alteromonas* ferment extract | Chelates Cadmium and Lead |

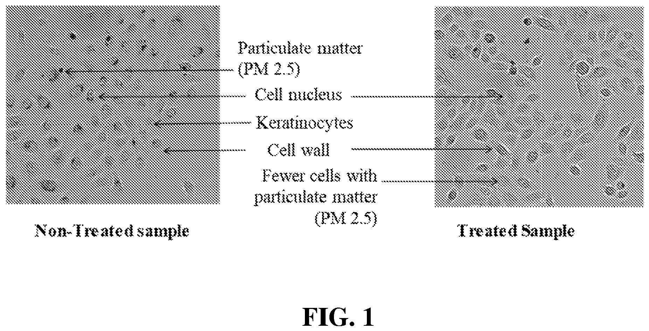

**Accumulation of PM 2.5 Fine Particles on Keratinocytes** - Human Keratinocytes cell culture was exposed to PM 2.5 ((NIST: Diesel Particulate Matter 2975) for 24 hours. The first group of Keratinocytes cell samples was treated with *Myrothamnus flabellifolia* extract during the exposure. A second group of Keratinocytes cell samples was not treated with *Myrothamnus flabellifolia* extract. Samples were obtained from each group and prepared for microscopic observation. The images were shown in FIG. 1. FIG. 1 illustrates that in the Keratinocytes samples treated with *Myrothamnus flabellifolia* extract, significantly fewer cells were polluted by particulate matter (PM 2.5) than the Keratinocytes samples not treated with *Myrothamnus flabellifolia* extract. This result indicates that *Myrothamnusflabellifolia* extract is effective in blocking accumulation of fine particle matter on the skin cells.

**Inhibition of Tumor Necrosis Factor Alpha (TNF-$\alpha$)** - *Myrothamnus flabellifolia* extract and saccharide isomerate have been shown to inhibit TNF-$\alpha$ production in keratinocytes. TNF-$\alpha$ is the prototype ligand of the TNF superfamily. It is a pleiotropic cytokine that plays a central role in inflammation. Increase in its expression is associated with an up regulation in pro-inflammatory activity. The bioassay used to analyze the effect of *Myrothamnus flabellifolia* extract and saccharide isomerate used a spectrophotometric measurement that reflects the presence of TNF-$\alpha$ and cellular viability. It was determined that *Myrothamnus flabellifolia* extract and saccharide isomerate inhibit TNF-$\alpha$ production in keratinocytes by 76% and 88%.

Subconfluent normal human adult keratinocytes (Cascade Biologics) cultivated in EPILIFE® standard growth medium (Cascade Biologics) at 37°C in 5% $CO_2$, were treated with phorbol 12-myristate 13-acetate (PMA, 10ng/ml, Sigma Chemical, #P1585-1MG) and either *Myrothamnus flabellifolia* extract and saccharide isomerate (treated samples) or no additional treatment (untreated sample) for 6 hours. PMA causes a dramatic increase in TNF-$\alpha$ secretion which peaks at 6 hours after treatment. Following incubation, cell culture medium was collected and the amount of TNF-a secretion quantified using a sandwich enzyme linked immuno-sorbant assay (ELISA) from R&D Systems (#DTA00C).

**Nitric Oxide Synthase Activity Assay:** Nitric oxide synthases (NOS) are a family of enzymes catalyzing the production of nitric oxide (NO) from L-arginine. NOS is an important mediator of vasodilation in blood vessels. This bioassay was used to analyze the effect of saccharide isomerate extract and *Myrothamnus flabellifolia* extract on the activity of NOS. The bioassay is performed in the presence or absence of the test compound, test extract, or positive control during production of NO by NOS. To measure the activity of NOS under, the NO product is allowed to oxidize to nitrite and nitrate and then the nitrate is reduced to nitrite and measured using an improved Griess method. It was shown that saccharide isomerate extract, and *Myrothamnus flabellifolia* extract reduced the activity of NOS greater than the 1 $\mu$M SIN-1 positive control. Specifically, the activity of NOS was reduced by approximately 45% by saccharide isomerate extract, and 36% by *Myrothamnusflabellifolia* extract. *See* Table 24.

**Production of Occludin-1:** Changes in the production of Occludin in keratinocytes due to saccharide isomerate extract was measured. Occludin-1 is a protein critical to the formulation of tight junctions and the skin's moisture barrier function. Occludin-1 production in treated and non-treated keratinocytes was determined by the use of a bioassay that analyzes Occludin-1 concentration in keratinocyte cell lysates. The bioassay was performed using PROTEINSIMPLE® SIMON™ western blotting protocol. For the samples, adult human epidermal keratinocytes (HEKa) from Life Technologies (C-005-5C) were grown at 37 °C and 5% CO2 for 24 hours in EPILIFE® growth media with calcium from Life Technologies (M-EP-500-CA) supplemented with Keratinocyte Growth Supplement (HKGS) from Life Technologies (S-101-5). HEKa were then incubated for 24 to 48 hours in growth medium containing test compound, no compound for negative control, or with 1mM CaCl$_2$ for positive control. The HEKa cells were then washed, collected, and stored on ice or colder until lysed on ice using a lysis buffer and sonication. The protein concentrations of the samples were determined and used to normalize the samples. The lysates were stored at -80 °C until use in the bioassay.

The PROTEINSIMPLE® SIMON™ western blotting bioassay assay employed a quantitative western blotting immunoassay technique using an antibody specific for Occludin to quantitatively detect Occludin in the test samples. Cell samples were lysed and normalized for protein concentration as described above. Normalized samples and molecular weight standards were loaded and ran on a denatured protein separation gel using capillary electrophoresis. The proteins in the gel were then immobilized and immunoprobed using a primary antibody specific for Occludin. The immobilized proteins were then immunoprobed with an enzyme-linked detection antibody that binds the primary antibody. A chemiluminescent substrate solution was then added to the immobilized proteins to allow chemiluminescent development in proportion to the amount of Occludin bound in the immobilization. The chemiluminescent development was stopped at a specific time and the intensity of the chemiluminescent signal was measured and compared to positive and negative controls. It was determined that saccharide isomerate increased keratinocyte production of Occludin-1 increased 171%. *See* table 24.

**Production of Filaggrin** - Saccharide isomerate has been shown to increase keratinocyte production of Filaggrin. Filaggrin is the precursor to Natural Moisturizing Factor (NMF) in the skin. Increased NMF increases the moisture content of the skin. Filaggrin production in treated and non-treated keratinocytes were determined using a bioassay that analyzes Filaggrin concentration in keratinocyte cell lysates. The bioassay was performed using PROTEINSIMPLE® Simon™ western blotting protocol. It was determined that saccharide isomerate increased keratinocyte production of Filaggrin by 197%.

For the samples, normal human epidermal keratinocytes (NHEK) were grown in EPI-200 -Mattek EPILIFE® growth media with calcium from Life Technologies (M-EP-500-CA). NHEK were incubated in growth medium overnight at 37 °C in 5% CO2 prior to treatment. NHEK were then incubated in growth medium with 1% test compound/extract or no compound/extract for 24 to 36 hours. The NHEK were then washed, collected, and stored on ice or colder until lysed on ice using a lysis buffer and sonication. The protein concentrations of the samples were determined and used to normalize the samples. The lysates were stored at -80 °C until use in the bioassay.

Briefly, the bioassay assay employs a quantitative western blotting immunoassay technique using an antibody specific for Filaggrin to quantitatively detect Filaggrin in the test samples. Cell samples were lysed and normalized for protein concentration. Normalized samples and molecular weight standards were then loaded and ran on a denatured protein separation gel using capillary electrophoresis. The proteins in the gel were immobilized and immunoprobed using a primary antibody specific for Filaggrin. The immobilized proteins were then immunoprobed with an enzyme-linked detection antibody that binds the primary antibody. A chemiluminescent substrate solution was then added to the immobilized proteins to allow chemiluminescent development in proportion to the amount of Filaggrin bound in the immobilization. The chemiluminescent development was stopped at a specific time and the intensity of the chemiluminescent signal was measured and compared to positive and negative controls.

**Heavy Metal Chelation:** *Alteromonas* ferment extract has been shown to chelate heavy metals. The biosorption capacity of EXO-P™ (i.e., 1% (10 g/L) of pure exopolysaccharide in butylene glycol with heavy metals cadmium and lead was evaluated. It was determined that *Alteromonas* ferment extract chelates heavy metals such as cadmium and lead.

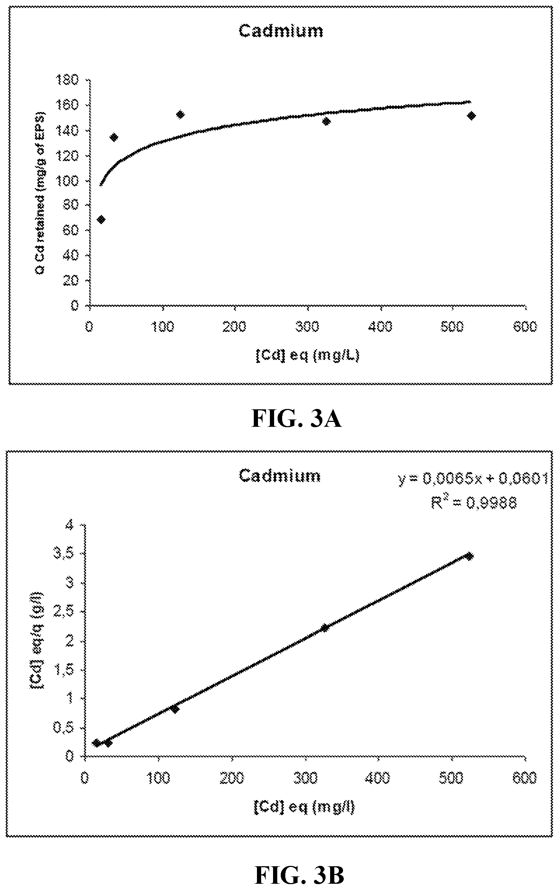

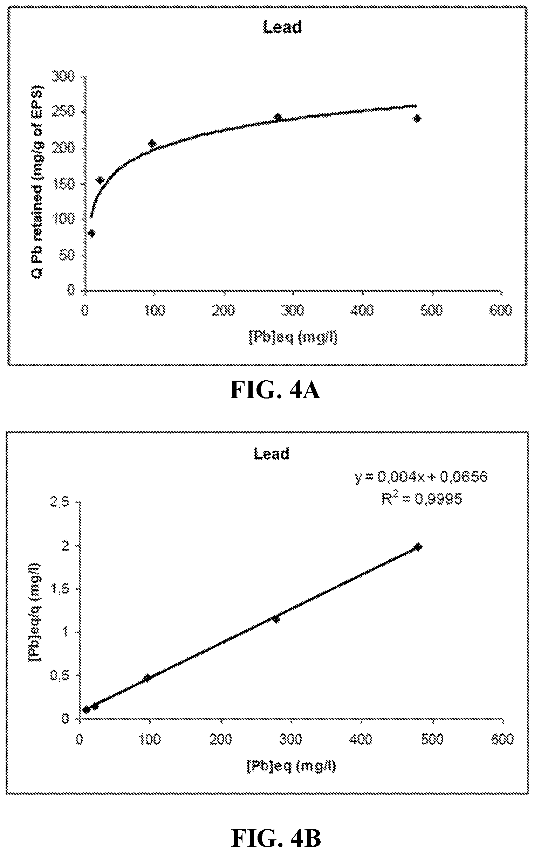

As shown in FIGs. 3A-3B, it was determined that cadmium retention by *Alteromonas* ferment extract (at isotherm) had a specific retention capacity $Q_{max}$ of 154 mg/g *Alteromonas* ferment extract exopolysaccharide (1.37 mmol/g of *Alteromonas* ferment extract exopolysaccharide) and a disassociation constant $K_d$ of 9.25 mg/L (0.08 mmol/L). As shown in FIGs. 4A-4B, it was determined that lead retention by *Alteromonas* ferment extract (at isotherm) had a specific retention capacity $Q_{max}$ of 250 mg/g *Alteromonas* ferment extract exopolysaccharide (1.21 mmol/g of *Alteromonas* ferment extract exopolysaccharide) and a disassociation *constant $K_d$* of 16.4 mg/L (0.08 mmol/L).

Briefly, the EXO-P™ was placed in contact with metallic cations (heavy metals) at a final concentration of 0.5 mg/mL for the exopolysaccharide *Alteromonas* ferment extract and 300 ppm (0.3 mg/mL) for the heavy metals, in a total volume of 30 mL in Erlenmeyer flasks. The pH was adjusted to 6 and the Erlenmeyer flasks were then placed under agitation for 3 hours at 200 rpm at 25 °C. The solutions were then filtered using AMICON® ultra centrifugal filters (VIVASPIN® 20, Vivascience) with a 30 kDa threshold under centrifugation at 3,000 G. The heavy metal concentration was measured in the filtrate by atomic absorption spectroscopy and the methods were normalized depending on the metallic cations being measured. The retention capacity of the metal by the exopolysaccharide was quantified with the following equation:

$$q = \frac{(Ci - Ceq) \cdot V}{m}$$

where *q* represents the quantity of metal retained by the exopolysaccharide (in mg or mmol/g); *Ci* represents the initial metal concentration; *Ceq* represents the metal concentration at equilibrium; *V* represents the volume of the solution (e.g., 50 mL); and *m* represents the exopolysaccharide (e.g., *Alteromonas* ferment extract) mass.

As shown in FIGs. 3A and 4A, the curves obtained for cadmium and lead show an increase in retention (expansion) at isotherm ending with a plateau, indicating that the exopolysaccharide's retention sites may have been approaching saturation with the respective metals. The model used applies the Langmuir model, which is characterized by the following equation:

$$q = \frac{(Ceq \cdot Qmax)}{(Kd + Ceq)}$$

where *Qmax* represents the specific retention capacity of the biosorbent (exopolysaccharide) at a saturation level; and *Kd* represents the dissociation constant of the metal with the biosorbent (exopolysaccharide).

The linear transformation of the Langmuir equation leads to the following equation:

$$\frac{Ceq}{q} = \frac{Kd}{Qmax} + \frac{Ceq}{Qmax}$$

As shown in FIGs. 3B and 4B, the linear charts quantify chelation with the *Qmax* and *Kd* parameters. A summary of quantitative results of the heavy metal chelation testing is found in Table 30 below. The *Alteromonas* ferment extract exopolysaccharide showed biosorption (chelation) capacities towards cadmium and lead. The commercial version of *Alteromonas* ferment extract (EXO-P™) is concentrated at 1% (10 g/L) with pure *Alteromonas* ferment extract expolysaccharide. Thus, a 1% (v/v) of EXO-P™ solution corresponds to a final exopolysaccharide *Alteromonas* ferment extract concentration of 0.1 g/L. As shown in Table 30, the observed $K_d$ values are all lower than 0.1 g/L (0.01% or 100 mg/L), which indicates that at a concentration of 0.1 g/L (0.01%) of EXO-P™ chelation of the metal by the exopolysaccharide is expected. Thus, chelation capacities of EXO-P™ for cadmium, and lead were effective from 0.01 to 0.04 g/L and higher.

**TABLE 30**

| Heavy Metal | $Q_{max}$ mg/g *Alteromonas* ferment extract (mmol/g) | $K_d$ mg/L (mmol/L) |
|---|---|---|
| Cadmium | 154 (1.37) | 9.25 (0.08) |
| Lead | 250 (1.21) | 16.4 (0.08) |

**EXAMPLE 6**

**(Clinical Efficacy of Cleanser and Freshener)**

**Cleansing Efficacy of Cleaning Particulate Matter:** Clinical efficacy of the cleansing effect of an embodiment of the cleanser containing a combination of *Alteromonas* ferment extract and *Myrothamnus flabellifolia* extract and an embodiment of the freshener formulation containing a combination of *Alteromonas* ferment extract and *Myrothamnus flabellifolia* extract as a follow up step was evaluated on 31 Asian females (average age: 40.65 +/- 8.79 years) using PM 2.5 ultraviolet fluorescent beads applied on the cheeks, as compared to water. It was determined that the cleanser cleaned the skin of fine particle matter better than cleansing with water alone. The freshener also showed an ability to further clean the skin of fine particle matter. A summary of quantitative results is found in Table 31 and the methods used to determine the properties of the ingredients are provided below.

**TABLE 31**

| Test Ingredients | Statistical measurement | Baseline | 15 minutes after application | Immediately After Cleanser Application |
|---|---|---|---|---|
| Cleanser - *Alteromonas* ferment extract and *Myrothamnus flabellifolia* extract | Mean | 3,823.76 | 156.29 | |
| | Percent Change* | | 95.91% | |
| | P-Value^ | | < 0.0001 | |
| Freshener - *Alteromonas* ferment extract and *Myrothamnus flabellifolia* extract | Mean | 156.29 | | 155.41 |
| | Percent Change* | | | 95.94% |
| | P-Value^ | | | |
| Water (Control) | Mean | 3,991.50 | 834.97 | |
| | Percent Change* | | 79.08% | |
| | P-Value^ | | < 0.0001 | |
| * Compared to baseline<br>^ Statistically significant when p-value ≤ 0.05 | | | | |

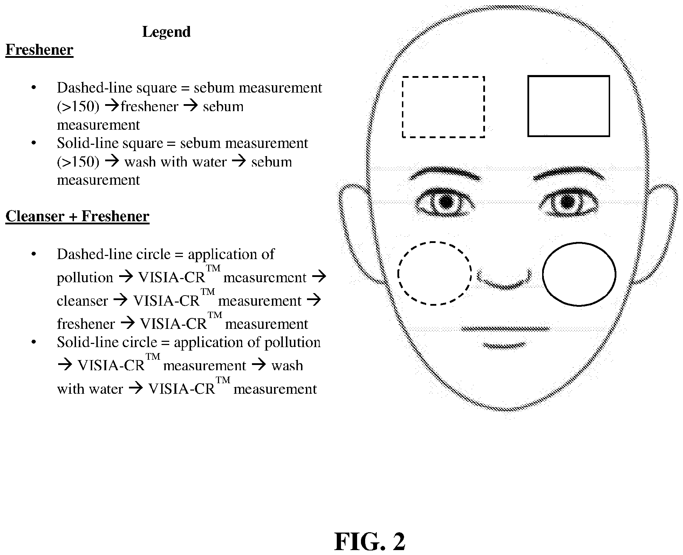

As shown in FIG. 2, the cheeks testing areas were marked for "Cleanser + Freshener," and water (control) application, per the randomization scheme to test efficacy of removing fine particle matter on the skin. The application of the PM 2.5 (FLUORESBRITE® Polychromatic Red Microspheres, Polysciences) material to all testing areas was used as the baseline. Images were captured after the PM 2.5 application via VISIA-CR™ (Canfield Imaging Systems, Fairfield NJ, USA) in Standard Light 1 and UV Filter Light. This was followed by imaging analyzing software to establish baseline.

The cleanser or water was then applied to the cheeks and wiped away with a warm, damp cotton towel. VISIA-CR™ image capturing and image analysis was performed after wiping the subject test area. Cleanser application and removal was then followed up with the freshener application and wiped away with a cotton ball. VISIA-CR™ and image analysis was then used as a final measure for the evaluation of the "Cleanser + Freshener" application.

Statistical analysis was conducted using the SPSS® software program (IBM, USA). To determine whether variables followed a normal distribution, the Shapiro-Wilks test was used. Statistical analysis of variables for parametric was conducted using the repeated measures ANOVA. If the value was non-parametric, all of them were initially compared by the Wilcoxon signed-rank test (Bonferroni correction was used to counteract the problem of multiple comparisons). A statistically significant difference was set at p-value ≤ 0.05. The results indicated that a combination of *Alteromonas* ferment extract and *Myrothamnus flabellifolia* extract is effective in cleansing the skin of accumulation of fine particle matter on the skin cells.

**Cleansing Efficacy of Removing Excess Sebum:** Clinical efficacy of an embodiment of the freshener formulation containing a combination of *Alteromonas* ferment extract and *Myrothamnusflabellifolia* extract on the removal of excess sebum on the forehead of thirty (30) Asian female subjects with an excessive forehead sebum measured at greater than 150 $\mu$g/mm$^2$ via Sebumeter SM815 (C+K, Germany) was evaluated, as compared to water. The subjects' faces were segmented in four (4) testing areas, as shown in FIG. 2. The forehead testing areas were marked for freshener application and water (control) application to remove excess sebum. All testing measurements were conducted in a temperature and humidity controlled room (22 +/- 2°C and 50 +/- 5%). It was determined that the cleansing efficacy of removing excess sebum was 19.2% greater than cleansing with water alone. A summary of quantitative results is found in Table 32 and the methods used to determine the properties of the ingredients are provided below.

**TABLE 32**

| Test Ingredients | Statistical measurement | Baseline | Immediately After Cleanser Application |
|---|---|---|---|
| Freshener - *Alteromonas* ferment extract and *Myrothamnus flabellifolia* extract | Mean | 166.16 | 70.65 |
| | Percent Change* | | -57.48% |
| | P-Value^ | | <0.0001 |
| Water (Control) | Mean | 169.03 | 90.52 |
| | Percent Change* | | -46.45% |
| | P-Value^ | | <0.0001 |
| * Compared to baseline ^ Statistically significant when p-value ≤ 0.05 | | | |

At baseline, testing areas were marked, per randomization scheme, on the forehead for the freshener application and water (control) application. The Sebumeter SM815 was used prior to the freshener and water application to establish an excess sebum baseline. After baseline was established, the freshener was applied to the forehead, along with water and wiped away with a cotton ball. Immediately after wiping away, the Sebumeter SM815 was used to record the reading. The results indicated that a combination of *Alteromonas* ferment extract and *Myrothamnusflabellifolia* extract are effective in removing excess sebum of the skin.

## EXAMPLE 7

### (Clinical Efficacy of Serum)

Clinical efficacy of the serum formulation also disclosed herein containing a combination of *Myrothamnus flabellifolia* extract, saccharide isomerate, and *Opuntia tuna* fruit extract was evaluated on the effectiveness to reduce the appearance of fine lines and wrinkles, reduce the appearance of skin yellowness, reduce the visibility of pores, reduce the appearance of skin dullness, improve the appearance of skin texture and/or roughness, reduce hyperpigmentation, reduce the appearance of skin redness, improve the appearance of skin tone, improve overall skin appearance (e.g., from photodamage), and improve skin barrier function. A summary of quantitative results compared to baseline is found in Tables 33 and 34 below and the methods used to determine the properties of the ingredients are provided in Table 35 below.

**TABLE 33**

| Activity | Statistical measurement | Baseline | % change after 2 weeks compared to baseline | % change after 4 weeks compared to baseline |
|---|---|---|---|---|
| Transepidermal water loss (TEWL) | Mean | 30.17 | 25.06 | 23.58 |
| | Percent change* | | -16.94% | -21.82% |
| | P-value^ | | <0.001 | <0.001 |
| Chroma Meter brightness (L*) | Mean | 58.85 | 59.66 | 60.41 |
| | Percent change* | | 1.38% | 2.65% |
| | P-value^ | | <0.001 | <0.001 |

(continued)

| Activity | Statistical measurement | Baseline | % change after 2 weeks compared to baseline | % change after 4 weeks compared to baseline |
|---|---|---|---|---|
| Chroma Meter brightness (a*) | Mean | 15.23 | 15.00 | 14.03 |
| | Percent change* | | -1.48% | -7.83% |
| | P-value^ | | NS | <0.001 |
| Chroma Meter brightness (b*) | Mean | 14.64 | 14.47 | 14.51 |
| | Percent change* | | -1.13% | -0.89% |
| | P-value^ | | NS | NS |
| * Compared to baseline<br>^ Statistically significant when $p$-value $\leq$ 0.05 | | | | |

**TABLE 34**

| Activity | Statistical measurement | Baseline | % change after 2 weeks compared to baseline | % change after 4 weeks compared to baseline |
|---|---|---|---|---|
| Reduction in appearance of fine lines and wrinkles | Mean | 4.70 | 4.63 | 4.35 |
| | Percent change* | | -1.42% | -7.45% |
| | P-value^ | | NS | <0.001 |
| Reduction in pore visibility | Mean | 5.13 | 5.03 | 4.45 |
| | Percent change* | | -1.95% | -13.31% |
| | P-value^ | | NS | <0.001 |
| Reduction in appearance of skin dullness | Mean | 5.92 | 5.67 | 5.38 |
| | Percent change* | | -4.23% | -9.01% |
| | P-value^ | | <0.001 | <0.001 |
| Improving skin tone | Mean | 5.18 | 5.18 | 4.95 |
| | Percent change* | | 0.00% | -4.50% |
| | P-value^ | | NS | 0.003 |
| Reduction in appearance of yellowness / sallowness | Mean | 5.52 | 5.38 | 5.12 |
| | Percent change* | | -2.42% | -7.25% |
| | P-value^ | | 0.008 | <0.001 |
| Reduce hyperpigmentation | Mean | 5.18 | 5.15 | 4.90 |
| | Percent change* | | -0.64% | -5.47% |
| | P-value^ | | NS | <0.001 |
| Improving skin texture / reducing roughness | Mean | 4.77 | 4.42 | 3.27 |
| | Percent change* | | -7.34% | -31.47% |
| | P-value^ | | <0.001 | <0.001 |

(continued)

| Activity | Statistical measurement | Baseline | % change after 2 weeks compared to baseline | % change after 4 weeks compared to baseline |
|---|---|---|---|---|
| Improving overall appearance / photodamage | Mean | 5.45 | 5.45 | 5.08 |
| | Percent change* | | 0.00% | -6.73% |
| | P-value^ | | NS | <0.001 |
| * Compared to baseline<br>^ Statistically significant when *p*-value ≤ 0.05 | | | | |

Recruitment criteria for the study included the following: thirty-five (35) Asian female subjects (ages 25-55 years old), with a minimum of 30 subjects completing the study; visible dull skin; visible hyperpigmentation; uneven skin tone; full face fine lines and wrinkles; visible yellowness and/or sallowness; visible redness; and visible texture and/or roughness.

Recruited subjects arrived to the testing facility five (5) days prior to the baseline date and started the five (5) day washout period with the provided neutral soap (NEUTROGENA®) to wash their face and supplemental sunscreen for daily use. Subjects were informed of the prohibited personal care products (i.e., creams, lotions, serum, facial treatment products) used on the face for the study duration with the exception of those provided. Subjects were further instructed to provide the testing site with any personal care products they currently use to evaluate and further prohibit all anti-aging products and instructed to stop use of those products.

Subjects returned to the testing site five (5) days after the start of the washout period. On arrival, the subjects were instructed to cleanse their face with the neutral soap and gently pat dry with a paper towel. After the cleansing, the subjects acclimated to the temperature and humidity controlled testing room (20-24°F and 30-50%) for twenty (20) minutes.

Following acclimation, subjects had the following procedures and measurements performed on their right cheek (Site 1) and left cheek (Site 2): Transepidermal Water Loss (TEWL); Chroma Meter (Konica Minolta) measurements at Sites 1 and 2; as well as live clinical grading of the following attributes: fine lines and wrinkles, yellowness/sallowness, pore visibility, dullness, roughness, hyperpigmentation, redness evenness of skin tone, and overall skin appearance/photo damage. The AQUAFLUX (closed chamber) was used to assess TEWL at baseline, Week 2 and Week 4 time points. The Chroma Meter (Konica Minolta) was used to assess skin color changes (black-white (L*), green-red (a*), blue-yellow (b*)) at baseline, Week 2, Week 4 time points. The Expert Clinical Grader was used to evaluate the entirety of the face (global) for all attributes listed above at baseline, Week 2 and Week 4 time points. After baseline, the subjects were then given the test product and instructed to apply the product in the AM and the PM. The subjects then returned 2 weeks and 4 weeks (±3 days) post-treatment, and the above procedure and measurements were repeated at each visit. Test products were weighed for compliance at each visit and subjects were instructed to not use the test product on the day of their scheduled visit. After the 4 week post-treatment visit, the subjects were instructed to return all testing products.

Statistical analyses tested the hypothesis that the pre-treatment values of each parameter were statistically different from post-treatment values. Statistical significance was declared if the two-tailed p-value was ≤ 0.05. The null hypothesis, that the mean change from baseline is zero, was tested using methods described in the Statistical Analysis Plan (Table 35) below. Statistical analyses were performed using SigmaPlot.

As shown in Table 33, the results indicated that, compared to baseline, *Myrothamnus flabellifolia* extract, saccharide isomerate, and *Opuntia tuna* fruit extract were effective in reducing transepidermal water loss (TEWL) by 16.9% after 2 weeks of use and 21.8% after 4 weeks of use; increasing brightness (L*) of the black-white color spectrum by 1.38% after 2 weeks of use and 2.65% after 4 weeks of use; decreasing brightness (a*) of the green-red color spectrum by 1.48% after 2 weeks of use and 7.83% after 4 weeks of use, and decreasing brightness (b*) of the blue-yellow spectrum by 1.13% after 2 weeks of use and 0.89% after 4 weeks of use.

**TABLE 35**

| Evaluation | Change from Baseline | Interpretation |
|---|---|---|
| Clinical grading of efficacy parameters | Paired t-test; if normality fails, a Wilcoxon signed rank test was performed | A *decrease* in scores indicates an improvement for the indicated parameter |

(continued)

| Evaluation | Change from Baseline | Interpretation |
|---|---|---|
| Transepidermal water loss (TEWL) | Paired t-test; if normality fails, a Wilcoxon signed rank test was performed | A *decrease* in TEWL indicates an improvement for the indicated parameter |
| Chroma Meter | Paired t-test; if normality fails, a Wilcoxon signed rank test was performed | An *increase* in L* indicates an improvement for the indicated parameter;<br><br>A *decrease* in a* and b* indicates an improvement for the indicated parameter |

**EXAMPLE 8**

**(Clinical Efficacy of Moisturizer)**

Clinical efficacy of the moisturizer formulation also disclosed herein containing a combination of *Myrothamnus flabellifolia* extract and saccharide isomerate was evaluated on the effectiveness to improve skin hydration. A summary of quantitative results compared to baseline is found in Table 36 below and the methods used to determine the properties of the ingredients are provided below.

**TABLE 36**

| Activity | Statistical Measurement | Baseline | Percent change after 15 minutes post-application | Percent change after 8 hours post-application |
|---|---|---|---|---|
| Improvement in skin hydration | Mean | 38.34 | 59.92 | 48.79 |
| | Percent Change* | | +56.3% | +27.3% |
| | P-value^ | | <0.001 | <0.001 |
| Untreated (control) | Mean | 37.91 | 37.90 | 38.04 |
| | Percent Change* | | -0.04% | 0.32% |
| | P-value^ | | NS | NS |
| * Compared to baseline<br>^ Statistically significant when *p*-value $\leq$ 0.05 | | | | |

Thirty (30) Asian female subjects (ages 18-65 years old) with a moisture measurement of 40 $\pm$ 10 a.u. via CORNEOMETER® CM 825 (Courage and Khazaka, Germany), were recruited with a minimum of twenty-five (25) subjects completing the study. A double-blinded randomized controlled study was conducted and included two (2) time-points: 15 minutes after application of an embodiment of the moisturizer and 8-hours after application of an embodiment of the moisturizer.

Three (3) days prior to the start of the study, enrolled subjects began a washout period on the forearm. Subjects received a neutral soap bar (NEUTROGENA®) to use for cleansing their forearms (i.e., bathing) for the washout period of three (3) days. Subjects received specific instructions prohibiting use of all personal care products (i.e., lotions, creams) on the test sites (e.g., volar forearm) for the entire washout and study duration except for the testing product. Following the washout period, subjects returned to the facility for baseline measurements.

The volar forearm was wiped with a damp disposable washcloth and pat dried with a paper towel. Two (2) test sites were marked on the volar forearm (one per randomization scheme). Each test site was 4 cm x 4 cm and the test sites were placed at least 2 cm from the wrist joint and 2 cm from the elbow joint. Measurements were made after the subjects acclimated to the temperature and humidity controlled testing room (22 $\pm$ 2°C and 40 $\pm$ 10% humidity) for 15 minutes.

Approximately 2 mg/cm$^2$ of the test product was applied to the test site and the other site was left untreated to serve as the negative control. After 15 minutes ($\pm$ 3 minutes) moisture measurements via CORNEOMETER® CM 825 (Courage and Khazaka, Germany) were made again for the immediate time-point (15 minutes post application). After the immediate measurement, subjects waited in a temperature and humidity controlled room for 7 hours and 45 minutes ± 10 minutes. The subjects then acclimated to the room again for 15 minutes and moisture measurements were taken 8 hours ± 10 minutes post product application. Statistical analyses tested the hypothesis that post-treatment values of the test parameters were statistically different from its pre-treatment and control values. Statistical significance was declared when the two-tailed p-value was ≤ 0.05.

The results indicated that, compared to baseline, a combination of *Myrothamnus flabellifolia* extract and saccharide isomerate were effective in increasing skin moisture immediately within 15 minutes after application of the moisturizer and provided continuous and skin hydration throughout the day after 8 hours of application of the moisturizer.

**EXAMPLE 9**

**(Assays that Can be Used to Test Compositions)**

Assays that can be used to determine the efficacy of any one of the ingredients or any combination of ingredients or compositions having said combination of ingredients disclosed throughout the specification and claims can be determined by methods known to those of ordinary skill in the art. The following are non-limiting assays that can be used in the context of the present invention. It should be recognized that other testing procedures can be used, including, for example, objective and subjective procedures.

**B16 Pigmentation Assay:** Melanogenesis is the process by which melanocytes produce melanin, a naturally produced pigment that imparts color to skin, hair, and eyes. Inhibiting melanogenesis is beneficial to prevent skin darkening and lighten dark spots associated with aging. This bioassay utilizes B16-F1 melanocytes (ATCC), an immortalized mouse melanoma cell line, to analyze the effect of compounds on melanogenesis. The endpoint of this assay is a spectrophotometric measurement of melanin production and cellular viability. B 16-F 1 melanocytes, can be cultivated in standard DMEM growth medium with 10% fetal bovine serum (Mediatech) at 37°C in 10% $CO_2$ and then treated with any one of the active ingredients, combination of ingredients, or compositions having said combinations disclosed in the specification for 6 days. Following incubation, melanin secretion is measured by absorbance at 405 nm and cellular viability is quantified.

**Collagen Stimulation Assay:** Collagen is an extracellular matrix protein critical for skin structure. Increased synthesis of collagen helps improve skin firmness and elasticity. This bioassay can be used to examine the effect of any one of the active ingredients, combination of ingredients, or compositions having said combinations disclosed in the specification on the production of procollagen peptide (a precursor to collagen) by human epidermal fibroblasts. The endpoint of this assay is a spectrophotometric measurement that reflects the presence of procollagen peptide and cellular viability. The assay employs the quantitative sandwich enzyme immunoassay technique whereby a monoclonal antibody specific for procollagen peptide has been pre-coated onto a microplate. Standards and samples can be pipetted into the wells and any procollagen peptide present is bound by the immobilized antibody. After washing away any unbound substances, an enzyme-linked polyclonal antibody specific for procollagen peptide can be added to the wells. Following a wash to remove any unbound antibody-enzyme reagent, a substrate solution can be added to the wells and color develops in proportion to the amount of procollagen peptide bound in the initial step using a microplate reader for detection at 450nm. The color development can be stopped and the intensity of the color can be measured. For generation of samples and controls, subconfluent normal human adult epidermal fibroblasts (Cascade Biologics) cultivated in standard DMEM growth medium with 10% fetal bovine serum (Mediatech) at 37°C in 10% $CO_2$, can be treated with each of the combination of ingredients or compositions having said combinations disclosed in the specification for 3 days. Following incubation, cell culture medium can be collected and the amount of procollagen peptide secretion quantified using a sandwich enzyme linked immuno-sorbant assay (ELISA) from Takara (#MK101).

**Elastin Stimulation Assay:** Elastin is a connective tissue protein that helps skin resume shape after stretching or contracting. Elastin is also an important load-bearing protein used in places where mechanical energy is required to be stored. Elastin is made by linking many soluble tropoelastin protein molecules, in a reaction catalyzed by lysyl oxidase. Elastin secretion and elastin fibers can be monitored in cultured human fibroblasts by staining of cultured human fibroblasts using immunofluorescent antibodies directed against elastin.

**Laminin Stimulation Assay:** Laminin and fibronectin are major proteins in the dermal-epidermal junction (DEJ) (also referred to as the basement membrane). The DEJ is located between the dermis and the epidermis interlocks forming fingerlike projections called rete ridges. The cells of the epidermis receive their nutrients from the blood vessels in the dermis. The rete ridges increase the surface area of the epidermis that is exposed to these blood vessels and the needed nutrients. The DEJ provides adhesion of the two tissue compartments and governs the structural integrity of the skin. Laminin and fibronectin are two structural glycoproteins located in the DEJ. Considered the glue that holds the cells together, laminin and fibronectin are secreted by dermal fibroblasts to help facilitate intra- and inter-cellular adhesion of the epidermal calls to the DEJ. Laminin secretion can be monitored by quantifying laminin in cell supernatants of cultured human fibroblasts treated for 3 days with culture medium with or without 1.0% final concentration of the test ingredient(s). Following incubation, laminin content can be measured using immunofluorescent antibodies directed against laminin in an enzyme linked immuno-sorbant assay (ELISA). Measurements are normalized for cellular metabolic activity, as determined by bioconversion of 3-(4,5-dimethylthiazol-2-yl)-5-(3-carboxymethoxyphenyl)-2-(4-sulfophenyl)-2H-tetrazolium (MTS).

**Tumor Necrosis Factor Alpha (TNF-$\alpha$) Assay:** The prototype ligand of the TNF superfamily, TNF-$\alpha$, is a pleiotropic cytokine that plays a central role in inflammation. Increase in its expression is associated with an up regulation in pro-inflammatory activity. This bioassay can be used to analyze the effect of any one of the active ingredients, combination of ingredients, or compositions having said combinations disclosed in the specification on the production of TNF-$\alpha$ by human epidermal keratinocytes. The endpoint of this assay can be a spectrophotometric measurement that reflects the presence of TNF-$\alpha$ and cellular viability. The assay employs the quantitative sandwich enzyme immunoassay technique whereby a monoclonal antibody specific for TNF-$\alpha$ has been pre-coated onto a microplate. Standards and samples can be pipetted into the wells and any TNF-$\alpha$ present is bound by the immobilized antibody. After washing away any unbound substances, an enzyme-linked polyclonal antibody specific for TNF-$\alpha$ can be added to the wells. Following a wash to remove any unbound antibody-enzyme reagent, a substrate solution can be added to the wells and color develops in proportion to the amount of TNF-$\alpha$ bound in the initial step using a microplate reader for detection at 450nm. The color development can be stopped and the intensity of the color can be measured. Subconfluent normal human adult keratinocytes (Cascade Biologics) cultivated in EPILIFE® standard growth medium (Cascade Biologics) at 37°C in 5% $CO_2$, can be treated with phorbol 12-myristate 13-acetate (PMA, 10ng/ml, Sigma Chemical, #P1585-1MG) and any one of the active ingredients, combination of ingredients, or compositions having said combinations disclosed in the specification for 6 hours. PMA has been shown to cause a dramatic increase in TNF-$\alpha$ secretion which peaks at 6 hours after treatment. Following incubation, cell culture medium can be collected and the amount of TNF-a secretion quantified using a sandwich enzyme linked immuno-sorbant assay (ELISA) from R&D Systems (#DTA00C).

**Antioxidant (AO) Assay:** An *in vitro* bioassay that measures the total anti-oxidant capacity of any one of the ingredients, combination of ingredients, or compositions having said combinations disclosed in the specification. The assay relies on the ability of antioxidants in the sample to inhibit the oxidation of ABTS® (2,2'-azino-di-[3-ethylbenzthiazoline sulphonate]) to ABTS®· + by metmyoglobin. The antioxidant system of living organisms includes enzymes such as superoxide dismutase, catalase, and glutathione peroxidase; macromolecules such as albumin, ceruloplasmin, and ferritin; and an array of small molecules, including ascorbic acid, $\alpha$-tocopherol, $\beta$-carotene, reduced glutathione, uric acid, and bilirubin. The sum of endogenous and food-derived antioxidants represents the total antioxidant activity of the extracellular fluid. Cooperation of all the different antioxidants provides greater protection against attack by reactive oxygen or nitrogen radicals, than any single compound alone. Thus, the overall antioxidant capacity may give more relevant biological information compared to that obtained by the measurement of individual components, as it considers the cumulative effect of all antioxidants present in plasma and body fluids. The capacity of the antioxidants in the sample to prevent ABTS oxidation is compared with that of Trolox, a water-soluble tocopherol analogue, and is quantified as molar Trolox equivalents. Anti-Oxidant capacity kit # 709001 from Cayman Chemical (Ann Arbor, Michigan USA) can be used as an *in vitro* bioassay to measure the total anti-oxidant capacity of each of any one of the active ingredients, combination of ingredients, or compositions having said combinations disclosed in the specification. The protocol can be followed according to manufacturer recommendations. The assay relied on antioxidants in the sample to inhibit the oxidation of ABTS® (2,2'-azino-di-[3-ethylbenzthiazoline sulphonate]) to ABTS®· + by metmyoglobin. The capacity of the antioxidants in the sample to prevent ABTS oxidation can be compared with that Trolox, a water-soluble tocopherol analogue, and can be quantified as a molar Trolox equivalent.

**ORAC Assay:** Oxygen Radical Absorption (or Absorbance) Capacity (ORAC) of any one of the active ingredients, combination of ingredients, or compositions having said combinations disclosed in the specification can also be assayed by measuring the antioxidant activity of such ingredients or compositions. Antioxidant activity indicates a capability to reduce oxidizing agents (oxidants). This assay quantifies the degree and length of time it takes to inhibit the action of an oxidizing agent, such as oxygen radicals, that are known to cause damage to cells (e.g., skin cells). The ORAC value of any one of the active ingredients, combination of ingredients, or compositions having said combinations disclosed in the specification can be determined by methods known to those of ordinary skill in the art (*see* U.S. Publication Nos. 2004/0109905 and 2005/0163880; and commercially available kits such as Zen-Bio ORAC Anti-oxidant Assay kit (#AOX-2)). The Zen-Bio ORAC Anti-oxidant Assay kit measures the loss of fluorescein fluorescence over time due to the peroxyl-radical formation by the breakdown of AAPH (2,2'-axobis-2-methyl propanimidamide, dihydrochloride). Trolox, a water soluble vitamin E analog, serves as positive control inhibition fluorescein decay in a dose dependent manner.

**Mushroom tyrosinase activity assay:** In mammalian cells, tyrosinase catalyzes two steps in the multi-step biosynthesis of melanin pigments from tyrosine (and from the polymerization of dopachrome). Tyrosinase is localized in melanocytes and produces melanin (aromatic quinone compounds) that imparts color to skin, hair, and eyes. Purified mushroom tyrosinase (Sigma) can be incubated with its substrate L-Dopa (Fisher) in the presence or absence of each of the active ingredients, any one of the combination of ingredients, or compositions having said combinations disclosed in the specification. Pigment formation can be evaluated by colorimetric plate reading at 490nm. The percent inhibition of mushroom tyrosinase activity can be calculated compared to non-treated controls to determine the ability of test ingredients or combinations thereof to inhibit the activity of purified enzyme. Test extract inhibition was compared with that of kojic acid (Sigma).

**Matrix Metalloproteinase 3 and 9 Enzyme Activity (MMP3; MMP9) Assay:** An *in vitro* matrix metalloprotease (MMP) inhibition assay. MMPs are extracellular proteases that play a role in many normal and disease states by virtue of their broad substrate specificity. MMP3 substrates include collagens, fibronectins, and laminin; while MMP9 substrates include collagen VII, fibronectins and laminin. Using Colorimetric Drug Discovery kits from BioMol International for MMP3 (AK-400) and MMP-9 (AK-410), this assay is designed to measure protease activity of MMPs using a thiopeptide as a chromogenic substrate (Ac-PLG-[2-mercapto-4-methyl-pentanoyl]-LG-OC2H5)5,6. The MMP cleavage site peptide bond is replaced by a thioester bond in the thiopeptide. Hydrolysis of this bond by an MMP produces a sulfhydryl group, which reacts with DTNB [5,5'-dithiobis(2- nitrobenzoic acid), Ellman's reagent] to form 2-nitro-5- thiobenzoic acid, which can be detected by its absorbance at 412 nm ($\epsilon$=13,600 M-1cm-1 at pH 6.0 and above 7). The active ingredients, any one of the combination of ingredients, or compositions having said combinations disclosed in the specification can be assayed.

**Matrix Metalloproteinase 1 Enzyme Activity (MMP1) Assay:** An *in vitro* matrix metalloprotease (MMP) inhibition assay. MMPs are extracellular proteases that play a role in many normal and disease states by virtue of their broad substrate specificity. MMP1 substrates include collagen IV. The Molecular Probes Enz/Chek Gelatinase/ Collagenase Assay kit (#E12055) utilizes a fluorogenic gelatin substrate to detect MMP1 protease activity. Upon proteolytic cleavage, bright green fluorescence is revealed and may be monitored using a fluorescent microplate reader to measure enzymatic activity.

The Enz/Chek Gelatinase/Collagenase Assay kit (#E12055) from Invitrogen is designed as an *in vitro* assay to measure MMP1 enzymatic activity. The active ingredients, any one of the combination of ingredients, or compositions having said combinations disclosed in the specification can be assayed. The assay relies upon the ability of purified MMP1 enzyme to degrade a fluorogenic gelatin substrate. Once the substrate is specifically cleaved by MMP1 bright green fluorescence is revealed and may be monitored using a fluorescent microplate reader. Test materials are incubated in the presence or absence of the purified enzyme and substrate to determine their protease inhibitor capacity.

**Cyclooxygenase (COX) Assay:** An *in vitro* cyclooxygenase-1 and -2 (COX-1, -2) inhibition assay. COX is a bifunctional enzyme exhibiting both cyclooxygenase and peroxidase activities. The cyclooxygenase activity converts arachidonic acid to a hydroperoxy endoperoxide (Prostaglandin G2; PGG2) and the peroxidase component reduces the endoperoxide (Prostaglandin H2; PGH2) to the corresponding alcohol, the precursor of prostaglandins, thromboxanes, and prostacyclins. This COX Inhibitor screening assay measures the peroxidase component of cyclooxygenases. The peroxidase activity is assayed colorimetrically by monitoring the appearance of oxidized N,N,N',N'-tetramethyl-p-phenylenediamine (TMPD). This inhibitor screening assay includes both COX-1 and COX-2 enzymes in order to screen isozyme-specific inhibitors. The Colormetric COX (ovine) Inhibitor screening assay (#760111, Cayman Chemical) can be used to analyze the effects of each of the active ingredients, any one of the combination of ingredients, or compositions having said combinations disclosed in the specification on the activity of purified cyclooxygnase enzyme (COX-1 or COX-2). According to manufacturer instructions, purified enzyme, heme and test extracts can be mixed in assay buffer and incubated with shaking for 15 min at room temperature. Following incubation, arachidonic acid and colorimetric substrate can be added to initiate the reaction. Color progression can be evaluated by colorimetric plate reading at 590nm. The percent inhibition of COX-1 or COX-2 activity can be calculated compared to non-treated controls to determine the ability of test extracts to inhibit the activity of purified enzyme.

**Lipoxygenase (LO) Assay:** An *in vitro* lipoxygenase (LO) inhibition assay. LOs are non-heme iron-containing dioxygenases that catalyze the addition of molecular oxygen to fatty acids. Linoleate and arachidonate are the main substrates for LOs in plants and animals. Arachadonic acid may then be converted to hydroxyeicosotrienenoic (HETE) acid derivatives, that are subsequently converted to leukotrienes, potent inflammatory mediators. This assay provides an accurate and convenient method for screening lipoxygenase inhibitors by measuring the hydroperoxides generated from the incubation of a lipoxygenase (5-, 12-, or 15-LO) with arachidonic acid. The Colorimetric LO Inhibitor screening kit (#760700, Cayman Chemical) can be used to determine the ability of each of the active ingredients, any one of the combination of ingredients, or compositions having said combinations disclosed in the specification to inhibit enzyme activity. Purified 15-lipoxygenase and test ingredients can be mixed in assay buffer and incubated with shaking for 10 min at room temperature. Following incubation, arachidonic acid can be added to initiate the reaction and the mixtures can be incubated for an additional 10 min at room temperature. Colorimetric substrate can be added to terminate catalysis and color progression can be evaluated by fluorescence plate reading at 490nm. The percent inhibition of lipoxyganse activity can be calculated compared to non-treated controls to determine the ability of each of the active ingredients, any one of the combination of ingredients, or compositions having said combinations disclosed in the specification to inhibit the activity of purified enzyme.

**Elastase Assay:** ENZCHEK® Elastase Assay (Kit# E-12056) from Molecular Probes (Eugene, Oregon USA) can be used as an *in vitro* enzyme inhibition assay for measuring inhibition of elastase activity for each of the active ingredients, any one of the combination of ingredients, or compositions having said combinations disclosed in the specification. The ENZCHEK® kit contains soluble bovine neck ligament elastin that can be labeled with dye such that the conjugate's fluorescence can be quenched. The non-fluorescent substrate can be digested by elastase or other proteases to yield highly fluorescent fragments. The resulting increase in fluorescence can be monitored with a fluorescence microplate reader. Digestion products from the elastin substrate have absorption maxima at ~505 nm and fluorescence emission maxima at ~515 nm. The peptide, N-methoxysuccinyl-Ala-Ala-Pro-Val-chloromethyl ketone, can be used as a selective, collective inhibitor of elastase when utilizing the ENZCHEK® Elastase Assay Kit for screening for elastase inhibitors.

**Oil Control Assay:** An assay to measure reduction of sebum secretion from sebaceous glands and/or reduction of sebum production from sebaceous glands can be assayed by using standard techniques known to those having ordinary skill in the art. In one instance, the forehead can be used. Each of the active ingredients, any one of the combination of ingredients, or compositions having said combinations disclosed in the specification can be applied to one portion of the forehead once or twice daily for a set period of days (e.g., 1, 2, 3, 4, 5, 6, 7, 8, 9, 10, 11, 12, 13, 14, or more days), while another portion of the forehead is not treated with the composition. After the set period of days expires, then sebum secretion can be assayed by application of fine blotting paper to the treated and untreated forehead skin. This is done by first removing any sebum from the treated and untreated areas with moist and dry cloths. Blotting paper can then be applied to the treated and untreated areas of the forehead, and an elastic band can be placed around the forehead to gently press the blotting paper onto the skin. After 2 hours the blotting papers can be removed, allowed to dry and then transilluminated. Darker blotting paper correlates with more sebum secretion (or lighter blotting paper correlates with reduced sebum secretion.

**Erythema Assay:** An assay to measure the reduction of skin redness can be evaluated using a Minolta Chroma Meter. Skin erythema may be induced by applying a 0.2% solution of sodium dodecyl sulfate on the forearm of a subject. The area is protected by an occlusive patch for 24 hrs. After 24 hrs., the patch is removed and the irritation-induced redness can be assessed using the a* values of the Minolta Chroma Meter. The a* value measures changes in skin color in the red region. Immediately after reading, the area is treated with the active ingredients, any one of the combination of ingredients, or compositions having said combinations disclosed in the specification. Repeat measurements can be taken at regular intervals to determine the formula's ability to reduce redness and irritation.

**Skin Moisture/Hydration Assay:** Skin moisture/hydration benefits can be measured by using impedance measurements with the Nova Dermal Phase Meter. The impedance meter measures changes in skin moisture content. The outer layer of the skin has distinct electrical properties. When skin is dry it conducts electricity very poorly. As it becomes more hydrated increasing conductivity results. Consequently, changes in skin impedance (related to conductivity) can be used to assess changes in skin hydration. The unit can be calibrated according to instrument instructions for each testing day. A notation of temperature and relative humidity can also be made. Subjects can be evaluated as follows: prior to measurement they can equilibrate in a room with defined humidity (e.g., 30-50%) and temperature (e.g., 68-72°C). Three separate impedance readings can be taken on each side of the face, recorded, and averaged. The T5 setting can be used on the impedance meter which averages the impedance values of every five seconds application to the face. Changes can be reported with statistical variance and significance. Each of the active ingredients, any one of the combination of ingredients, or compositions having said combinations disclosed in the specification can be assayed according to this process.

**Skin Clarity and Reduction in Freckles and Age Spots Assay:** Skin clarity and the reduction in freckles and age spots can be evaluated using a Minolta Chroma Meter. Changes in skin color can be assessed to determine irritation potential due to product treatment using the a* values of the Minolta Chroma Meter. The a* value measures changes in skin color in the red region. This is used to determine whether each of the active ingredients, any one of the combination of ingredients, or compositions having said combinations disclosed in the specification is inducing irritation. The measurements can be made on each side of the face and averaged, as left and right facial values. Skin clarity can also be measured using the Minolta Meter. The measurement is a combination of the a*, b, and L values of the Minolta Meter and is related to skin brightness, and correlates well with skin smoothness and hydration. Skin reading is taken as above. In one non-limiting aspect, skin clarity can be described as L/C where C is chroma and is defined as $(a^2 + b^2)^{1/2}$.

**Skin Dryness, Surface Fine Lines, Skin Smoothness, and Skin Tone Assay:** Skin dryness, surface fine lines, skin smoothness, and skin tone can be evaluated with clinical grading techniques. For example, clinical grading of skin dryness can be determined by a five point standard Kligman Scale: (0) skin is soft and moist; (1) skin appears normal with no visible dryness; (2) skin feels slightly dry to the touch with no visible flaking; (3) skin feels dry, tough, and has a whitish appearance with some scaling; and (4) skin feels very dry, rough, and has a whitish appearance with scaling. Evaluations can be made independently by two clinicians and averaged.

**Clinical Grading of Skin Tone Assay:** Clinical grading of skin tone can be performed *via* a ten point analog numerical scale: (10) even skin of uniform, pinkish brown color. No dark, erythremic, or scaly patches upon examination with a hand held magnifying lens. Microtexture of the skin very uniform upon touch; (7) even skin tone observed without magnification. No scaly areas, but slight discolorations either due to pigmentation or erythema. No discolorations more than 1 cm in diameter; (4) both skin discoloration and uneven texture easily noticeable. Slight scaliness. Skin rough to the touch in some areas; and (1) uneven skin coloration and texture. Numerous areas of scaliness and discoloration, either hypopigmented, erythremic or dark spots. Large areas of uneven color more than 1 cm in diameter. Evaluations were made independently by two clinicians and averaged.

**Clinical Grading of Skin Smoothness Assay:** Clinical grading of skin smoothness can be analyzed *via* a ten point analog numerical scale: (10) smooth, skin is moist and glistening, no resistance upon dragging finger across surface; (7) somewhat smooth, slight resistance; (4) rough, visibly altered, friction upon rubbing; and (1) rough, flaky, uneven surface. Evaluations were made independently by two clinicians and averaged.

**Skin Smoothness and Wrinkle Reduction Assay With Methods Disclosed in Packman *et al.* (1978):** Skin smoothness and wrinkle reduction can also be assessed visually by using the methods disclosed in Packman *et al.* (1978). For example, at each subject visit, the depth, shallowness and the total number of superficial facial lines (SFLs) of each subject can be carefully scored and recorded. A numerical score was obtained by multiplying a number factor times a depth/width/length factor. Scores are obtained for the eye area and mouth area (left and right sides) and added together as the total wrinkle score.

**Skin Firmness Assay with a Hargens Ballistometer:** Skin firmness can be measured using a Hargens ballistometer, a device that evaluates the elasticity and firmness of the skin by dropping a small body onto the skin and recording its first two rebound peaks. The ballistometry is a small lightweight probe with a relatively blunt tip (4 square mm-contact area) was used. The probe penetrates slightly into the skin and results in measurements that are dependent upon the properties of the outer layers of the skin, including the stratum corneum and outer epidermis and some of the dermal layers.

**Skin Softness/Suppleness Assay with a Gas Bearing Electrodynamometer:** Skin softness/suppleness can be evaluated using the Gas Bearing Electrodynamometer, an instrument that measures the stress/strain properties of the skin. The viscoelastic properties of skin correlate with skin moisturization. Measurements can be obtained on the predetermined site on the cheek area by attaching the probe to the skin surface with double-stick tape. A force of approximately 3.5 gm can be applied parallel to the skin surface and the skin displacement is accurately measured. Skin suppleness can then be calculated and is expressed as DSR (Dynamic Spring Rate in gm/mm).

**Appearance of Lines and Wrinkles Assay with Replicas:** The appearance of lines and wrinkles on the skin can be evaluated using replicas, which is the impression of the skin's surface. Silicone rubber like material can be used. The replica can be analyzed by image analysis. Changes in the visibility of lines and wrinkles can be objectively quantified *via* the taking of silicon replicas form the subjects' face and analyzing the replicas image using a computer image analysis system. Replicas can be taken from the eye area and the neck area, and photographed with a digital camera using a low angle incidence lighting. The digital images can be analyzed with an image processing program and are of the replicas covered by wrinkles or fine lines was determined.

**Surface Contour of the Skin Assay with a Profilometer/Stylus Method:** The surface contour of the skin can be measured by using the profilometer/Stylus method. This includes either shining a light or dragging a stylus across the replica surface. The vertical displacement of the stylus can be fed into a computer *via* a distance transducer, and after scanning a fixed length of replica a cross-sectional analysis of skin profile can be generated as a two-dimensional curve. This scan can be repeated any number of times along a fix axis to generate a simulated 3-D picture of the skin. Ten random sections of the replicas using the stylus technique can be obtained and combined to generate average values. The values of interest include Ra which is the arithmetic mean of all roughness (height) values computed by integrating the profile height relative to the mean profile height. Rt which is the maximum vertical distance between the highest peak and lowest trough, and Rz which is the mean peak amplitude minus the mean peak height. Values are given as a calibrated value in mm. Equipment should be standardized prior to each use by scanning metal standards of know values. Ra Value can be computed by the following equation: $R_a$ = Standardize roughness; $l_m$ = the traverse (scan) length; and y = the absolute value of the location of the profile relative to the mean profile height (x-axis).

**MELANODERM™ Assay:** In other non-limiting aspects, the efficacy of each of the active ingredients, any one of the combination of ingredients, or compositions having said combinations disclosed in the specification can be evaluated by using a skin analog, such as, for example, MELANODERM™. Melanocytes, one of the cells in the skin analog, stain positively when exposed to L-dihydroxyphenyl alanine (L-DOPA), a precursor of melanin. The skin analog, MELANODERM™, can be treated with a variety of bases containing each of the active ingredients, any one of the combination of ingredients, or compositions having said combinations disclosed in the specification or with the base alone as a control. Alternatively, an untreated sample of the skin analog can be used as a control.

**Keratinocyte Monolayer Permeability** - Changes in the permeability of a keratinocyte monolayer due to each of the active ingredients, any one of the combination of ingredients, or compositions having said combinations disclosed in the specification can be measured. Keratinocyte monolayer permeability is a measure of skin barrier integrity. Keratinocyte monolayer permeability in treated and non-treated keratinocytes can be determined using, as a non-limiting example, the *In Vitro* Vascular Permeability assay by Millipore (ECM642). This assay analyzes endothelial cell adsorption, transport, and permeability. Briefly, adult human epidermal keratinocytes from Life Technologies (C-005-5C) can be seeded onto a porous collagen-coated membrane within a collection well. The keratinocytes are then incubated for 24 hours at 37 °C and 5% $CO_2$ in EPILIFE® growth media with calcium from Life Technologies (M-EP-500-CA) supplemented with Keratinocyte Growth Supplement (HKGS) from Life Technologies (S-101-5). This incubation time allows the cells to form a monolayer and occlude the membrane pores. The media is then replaced with fresh media with (test sample) or without (non-treated control) test compounds/extracts and the keratinocytes are incubated for an additional 48 hours at 37 °C and 5% $CO_2$. To determine permeability of the keratinocyte monolayer after incubation with/without the test compound/extract, the media is replaced with fresh media containing a high molecular weight Fluorescein isothiocyanate (FITC)-Dextran and the keratinocytes are incubated for 4 hours at 37 °C and 5% $CO_2$. During the 4 hours incubation, FITC can pass through the keratinocytes monolayer and porous membrane into the collection well at a rate proportional to the monolayer's permeability. After the 4 hour incubation, cell viability and the content of FITC in the collection wells can be determined. For the FITC content, the media in the collection well is collected and fluorescence of the media determined at 480nm (Em) when excited at 520nm. Percent permeability and percent change in comparison to the non-treated controls can be determined by the following equations: Percent Permeability = ((Mean Ex/Em of test sample)/Mean Ex/Em untreated control)* 100; Percent Change = Percent Permeability of test sample - Percent Permeability of untreated control.

**Production of Hyaluronic Acid** - Changes in the production of hyaluronic acid in human dermal fibroblasts due to each of the active ingredients, any one of the combination of ingredients, or compositions having said combinations disclosed in the specification can be measured. HA is a polysaccharide involved in stabilization of the structure of the matrix and is involved in providing turgor pressure to tissue and cells. As one non-limiting example, HA production in treated and non-treated adult human dermal fibroblasts (HDFa) cells can be determined using the Hyaluronan DuoSet ELISA kit from R&D Systems (DY3614). In this assay, for production of samples, subconfluent HDFa cells from Cascade Biologics (C-13-5C) are incubated at 37 °C and 10% $CO_2$ in starvation medium (0.15% fetal bovine serum and 1% Penicillin Streptomycin solution in Dulbecco's Modified Eagle Medium) for 72 hours prior to treatment. The cells are then incubated with fresh starvation medium with either test compound, positive control (phorbol 12-myristate 13-acetate from Sigma-Aldrich (P1585) and platelet derived growth factor from Sigma-Aldrich (P3201)), or no additive for 24 hours. Media is then collected and frozen at -80 °C until use in the ELISA assay.

Briefly, the ELISA assay employs a quantitative sandwich enzyme immunoassay technique whereby a capture antibody specific for HA can be pre-coated onto a microplate. Standards and media from treated and untreated cells are pipetted into the microplate wells to enable any HA present to be bound by the immobilized antibody. After washing away any unbound substances, an enzyme-linked detection antibody specific for HA is added to the wells. Following a wash to remove any unbound antibody-enzyme reagent, a substrate solution is added to the wells to allow color development in proportion to the amount of HA bound in the initial step. The color development is stopped at a specific time and the intensity of the color at 450nm can be measured using a microplate reader.

**Inhibition of Hyaluronidase Activity** - Changes in the activity of hyaluronidase due to each of the active ingredients, any one of the combination of ingredients, or compositions having said combinations disclosed in the specification can be measured. Hyaluronidase is an enzyme that degrades HA. HA is a polysaccharide involved in stabilization of the structure of the matrix and is involved in providing turgor pressure to tissue and cells. As one non-limiting example, hyaluronidase activity can be determined using an *in vitro* protocol modified from Sigma-Aldrich protocol # EC 3.2.1.35. Briefly, hyaluronidase type 1-S from Sigma-Aldrich (H3506) is added to microplate reaction wells containing test compound or controls. Tannic acid can be used as a positive control inhibitor, no test compound can be added for the control enzyme, and wells with test compound or positive control but without hyaluronidase can be used as a background negative control. The wells are incubated at 37 °C for 10 minutes before addition of substrate (HA). Substrate is added and the reactions incubated at 37 °C for 45 minutes. A portion of each reaction solution is then transferred to and gently mixed in a solution of sodium acetate and acetic acid pH 3.75 to stop that portion of the reaction (stopped wells). The stopped wells and the reaction wells should both contain the same volume of solution after addition of the portion of the reaction solution to the stopped wells. Both the reaction wells and the stopped wells are incubated for 10 minutes at room temperature. Absorbance at 600nm is then measured for both the reaction wells and the stopped wells. Inhibition can be calculated using the following formulas: Inhibitor (or control) activity = (Inhibitor stopped wells absorbance at 600nm - inhibitor reaction wells absorbance at 600nm); Initial activity = control enzyme absorbance at 600nm; Percent Inhibition = [(Initial activity/ Inhibitor Activity)*100]-100.

**Peroxisome Proliferator-Activated Receptor Gamma (PPAR-$\gamma$) Activity** - Changes in the activity of PPAR-$\gamma$ due to each of the active ingredients, any one of the combination of ingredients, or compositions having said combinations disclosed in the specification can be measured. PPAR-$\gamma$ is a receptor critical for the production of sebum. As one non-limiting example, the activity of PPAR-$\gamma$ can be determined using a bioassay that analyzes the ability of a test compound or composition to inhibit binding of a ligand. Briefly, fluorescent small-molecule pan-PPAR ligand, FLUORMONE™ Pan-PPAR Green, available from Life Technologies (PV4894), can be used to determine if test compounds or compositions are able to inhibit binding of the ligand to PPAR-$\gamma$. The samples wells include PPAR-$\gamma$ and fluorescent ligand and either: test compound or composition (test); a reference inhibitor, rosiglitazone (positive control); or no test compound (negative control). The wells are incubated for a set period of time to allow the ligand opportunity to bind the PPAR-$\gamma$. The fluo-

rescence polarization of each sample well can then be measured and compared to the negative control well to determine the percentage of inhibition by the test compound or composition.

All of the compositions and/or methods disclosed and claimed herein can be made and executed without undue experimentation in light of the present disclosure. More specifically, it will be apparent that certain agents which are both chemically and physiologically related may be substituted for the agents described herein while the same or similar results would be achieved.

## REFERENCES

Cosmetic Ingredient Dictionary, Third Edition, CTFA, 1982
International Cosmetic Ingredient Dictionary, Fourth edition, CTFA, 1991
International Cosmetic Ingredient Dictionary and Handbook, Tenth Edition, CTFA, 2004
International Cosmetic Ingredient Dictionary and Handbook, Twelfth Edition, CTFA, 2008

## Claims

1. A topical composition comprising:

   (a) an effective amount *of Myrothamnus flabellifolia* extract and *Alteromonas* ferment extract; and, optionally,
   (b) an effective amount of saccharide isomerate; and/or
   (c) *Opuntia tuna* fruit extract.

2. The composition of claim 1, further comprising sunscreen agent selected from the group of octinoxate, zinc oxide, ethylhexyl salicylate, ensulizole, homosalate, avobenzone, octocrylene, oxybenzone, and combinations thereof.

3. The composition of any of claims 1 to 2, wherein the composition comprises an effective amount of *Myrothamnus flabellifolia* extract and *Alteromonas* ferment extract to block accumulation of particulate matter with an aerodynamic diameter equal to or less than 2.5 $\mu$m on skin.

4. The composition of any of claims 1 to 3, wherein the *Myrothamnus flabellifolia* extract is an extract of the leaf and/or stem of *Myrothamnus flabellifolia,* the saccharide isomerate comprises an exopolysaccharide of *Vibrio alginolyticus* belonging to the family of Thalasso plankton and is an aqueous extract, and/or the *Alteromonas* ferment extract comprises an exopolysaccharide from Kopara and is an aqueous-alcoholic extract.

5. The composition of any of claims 1 to 4, wherein the composition is an emulsion, serum, gel, gel emulsion, or gel serum.

6. The composition of any of claims 1 to 5, wherein the composition is an oil-in-water emulsion or water-in-oil-emulsion.

7. The composition of any of claims 1 to 6, wherein the topical composition is formulated as at least one of a mask, serum, moisturizer, an eye gel, a facial emulsion, a freshener, and a cleanser.

8. A cosmetic method of improving a condition or appearance of skin comprising applying a topical composition as defined in any of claims 1 to 7, wherein the skin is treated to block the accumulation of fine particulate matter with an aerodynamic diameter equal to or less than 2.5 $\mu$m on the skin, and, optionally, inhibit nitric oxide synthase, and/or inhibit TNF-$\alpha$.

9. The method of claim 8, wherein the composition is applied to skin of a face.

## Patentansprüche

1. Topische Zusammensetzung, umfassend:

   (a) eine wirksame Menge von *Myrothammis flabellifolia*-Extrakt und Alteromonas-Ferment-Extrakt und gegeben falls

(b) eine wirksame Menge eines Saccharidisomerats und/oder

(c) *Opuntia tuna*-Frucht-Extrakt.

**2.** Zusammensetzung nach Anspruch 1, die ferner ein Sonnenschutzmittel enthält, das aus der Gruppe von Octinoxat, Zinkoxid, Ethylhexylsalicylat, Ensulizol, Homosalat, Avobenzon, Octocrylen, Oxybenzon und Kombinationen davon ausgewählt ist.

**3.** Zusammensetzung nach einem der Ansprüche 1 bis 2, wobei die Zusammensetzung eine wirksame Menge an *Myrothammis* flabellifolia-Extrakt und *Alteromonas* ferment-Extrakt enthält, um die Ansammlung von Partikeln mit einem aerodynamischen Durchmesser von 2,5 μm oder weniger auf der Haut zu verhindern.

**4.** Zusammensetzung nach einem der Ansprüche 1 bis 3, wobei der *Myrothammis* flabellifolia-Extrakt ein Extrakt des Blattes und/oder des Stammes von *Myrothammis flabellifolia* ist, das Saccharid-Isomerat ein Exopolysaccharid von *Vibrio alginolyticus,* das zur Familie des Thalasso-Planktons gehört, umfasst und ein wässriger Extrakt ist, und/oder der Alteromonas-Ferment-Extrakt ein Exopolysaccharid aus Kopara umfasst und ein wässrig-alkoholischer Extrakt ist.

**5.** Die Zusammensetzung nach einem der Ansprüche 1 bis 4, wobei die Zusammensetzung eine Emulsion, ein Serum, ein Gel, eine Gel-Emulsion oder ein Gel-Serum ist.

**6.** Die Zusammensetzung nach einem der Ansprüche 1 bis 5, wobei die Zusammensetzung eine Öl-in-Wasser-Emulsion oder Wasser-in-Öl-Emulsion ist.

**7.** Zusammensetzung nach einem der Ansprüche 1 bis 6, wobei die topische Zusammensetzung als mindestens eines der folgenden formuliert ist: Maske, Serum, Feuchtigkeitscreme, Augengel, Gesichtsemulsion, Auffrischungsmittel und Reinigungsmittel.

**8.** Kosmetisches Verfahren zur Verbesserung des Zustands oder des Aussehens der Haut, umfassend das Auftragen einer topischen Zusammensetzung, wie sie in einem der Ansprüche 1 bis 7 definiert ist, wobei die Haut behandelt wird, um die Ansammlung feiner Partikel mit einem aerodynamischen Durchmesser von 2,5 μm oder weniger auf der Haut zu blockieren, und gegebenenfalls die Stickoxid-Synthase zu hemmen und/oderTNF-α zu hemmen.

**9.** Verfahren nach Anspruch 8, wobei die Zusammensetzung auf die Haut eines Gesichts aufgetragen wird.

**Revendications**

**1.** Composition topique comprenant :

(a) une quantité efficace d'extrait de *Myrothamnus flabellifolia* et d'extrait de ferment *d'Alteromonas ;* et, facultativement,

(b) une quantité efficace d'isomérate de saccharide ; et/ou

(c) un extrait de fruit *d'Opuntia tuna.*

**2.** Composition selon la revendication 1, comprenant en outre un agent écran solaire sélectionné dans le groupe de l'octinoxate, de l'oxyde de zinc, du salicylate d'éthylhexyle, de l'ensulizole, de l'homosalate, de l'avobenzone, de l'octocrylène, de l'oxybenzone et des combinaisons de ceux-ci.

**3.** Composition selon l'une quelconque des revendications 1 à 2, dans laquelle la composition comprend une quantité efficace d'extrait de *Myrothamnus flabellifolia* et d'extrait de ferment *d'Alteromonas* pour bloquer l'accumulation de matières particulaires d'un diamètre aérodynamique inférieur ou égal à 2,5 μm sur la peau.

**4.** Composition selon l'une quelconque des revendications 1 à 3, dans laquelle l'extrait de *Myrothamnus flabellifolia* est un extrait de la feuille et/ou de la tige de *Myrothamnus flabellifolia,* l'isomérate de saccharide comprend un exopolysaccharide de *Vibrio alginolyticus* appartenant à la famille du plancton thermal et est un extrait aqueux, et/ou l'extrait de ferment *d'Alteromonas* comprend un exopolysaccharide de Kopara et est un extrait hydroalcoolique.

**5.** Composition selon l'une quelconque des revendications 1 à 4, dans laquelle la composition est une émulsion, un sérum, un gel, une émulsion en gel ou un sérum en gel.

**6.** Composition selon l'une quelconque des revendications 1 à 5, dans laquelle la composition est une émulsion huile dans l'eau ou une émulsion eau dans l'huile.

**7.** Composition selon l'une quelconque des revendications 1 à 6, dans laquelle la composition topique est formulée sous la forme d'au moins l'un d'un masque, d'un sérum, d'une crème hydratante, d'un gel pour les yeux, d'une émulsion pour le visage, d'une lotion rafraîchissante et d'un nettoyant.

**8.** Procédé cosmétique d'amélioration d'une affection cutanée ou d'un aspect de la peau comprenant l'application d'une composition topique selon l'une quelconque des revendications 1 à 7, dans lequel la peau est traitée pour bloquer l'accumulation de fines matières particulaires d'un diamètre aérodynamique inférieur ou égal à 2,5 $\mu$m sur la peau, et, facultativement, inhiber la synthase d'oxyde nitrique, et/ou inhiber le TNF-$\alpha$.

**9.** Procédé selon la revendication 8, dans lequel la composition est appliquée sur la peau d'un visage.

Particulate matter (PM 2.5)
Cell nucleus
Keratinocytes
Cell wall
Fewer cells with particulate matter (PM 2.5)

Non-Treated sample

Treated Sample

**FIG. 1**

**Legend**

**Freshener**

- Dashed-line square = sebum measurement (>150) →freshener → sebum measurement
- Solid-line square = sebum measurement (>150) → wash with water → sebum measurement

**Cleanser + Freshener**

- Dashed-line circle = application of pollution → VISIA-CR$^{TM}$ measurement → cleanser → VISIA-CR$^{TM}$ measurement → freshener → VISIA-CR$^{TM}$ measurement
- Solid-line circle = application of pollution → VISIA-CR$^{TM}$ measurement → wash with water → VISIA-CR$^{TM}$ measurement

**FIG. 2**

**FIG. 3A**

**FIG. 3B**

**FIG. 4A**

**FIG. 4B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62623309
- US 62745078 B
- US 2017020808 A1
- KR 20160068310 A
- US 5011681 A
- US 4421769 A
- US 3755560 A
- US 5087445 A
- US 4509949 A

- US 2798053 A
- US 5100660 A
- US 4849484 A
- US 4835206 A
- US 4628078 A
- US 4599379 A
- US 20040109905 A
- US 20050163880 A

**Non-patent literature cited in the description**

- International Cosmetic Ingredient Dictionary and Handbook. 2008, vol. 2, 1731
- CTFA Cosmetic Ingredient Dictionary
- CTFA International Cosmetic Ingredient Dictionary. 1991, 12, , 80
- Cosmetic Ingredient Dictionary. CTFA, 1982

- International Cosmetic Ingredient Dictionary. CTFA, 1991
- International Cosmetic Ingredient Dictionary and Handbook. CTFA, 2004
- International Cosmetic Ingredient Dictionary and Handbook. CTFA, 2008